(19) Europäisches Patentamt
European Patent Office
Office européen des brevets

(11) EP 4 077 730 B1

# (12) EUROPEAN PATENT SPECIFICATION

(45) Date of publication and mention of the grant of the patent:
**18.02.2026 Bulletin 2026/08**

(21) Application number: **20838519.5**

(22) Date of filing: **21.12.2020**

(51) International Patent Classification (IPC):
***C12Q 1/6883*** *(2018.01)* ***G01N 33/68*** *(2006.01)*

(52) Cooperative Patent Classification (CPC):
**C12Q 1/6883; G01N 33/6896;** C12Q 2600/112; C12Q 2600/158; C12Q 2600/178; G01N 2800/30; G01N 2800/50

(86) International application number:
**PCT/EP2020/087447**

(87) International publication number:
**WO 2021/123428 (24.06.2021 Gazette 2021/25)**

## (54) METHODS AND KITS FOR CLASSIFICATION OF PSYCHOTIC DISORDER SUBJECTS AND COMPOUNDS FOR USE IN THERAPY OF SUCH DISORDERS

VERFAHREN UND KITS ZUR KLASSIFIZIERUNG VON PATIENTEN MIT PSYCHOTISCHEN ERKRANKUNGEN UND VERBINDUNGEN ZUR BEHANDLUNG DER KRANKHEITEN

PROCÉDÉS ET KITS POUR LA CLASSIFICATION DE SUJETS SOUFFRANT DE TROUBLES PSYCHOTIQUES ET COMPOSÉS POUR LE TRAITEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2019 EP 19218841**

(43) Date of publication of application:
**26.10.2022 Bulletin 2022/43**

(73) Proprietor: **Centre Hospitalier Universitaire Vaudois (CHUV)**
**1011 Lausanne (CH)**

(72) Inventors:
- **DO CUENOD, Kim Q.**
  **1814 La Tour de Peilz (CH)**
- **KHADIMALLAH, Inès**
  **1010 Lausanne (CH)**

(74) Representative: **KATZAROV S.A.**
**Geneva Business Center**
**12 Avenue des Morgines**
**1213 Petit-Lancy (CH)**

(56) References cited:

| | |
|---|---|
| **US-A1- 2007 105 105** | **US-A1- 2007 105 105** |
| **US-A1- 2016 213 753** | **US-A1- 2016 213 753** |
| **US-B2- 8 163 475** | **US-B2- 8 163 475** |

- **MA JIE ET AL: "Identification of miR-22-3p, miR-92a-3p, and miR-137 in peripheral blood as biomarker for schizophrenia", PSYCHIATRY RESEARCH, ELSEVIER IRELAND LTD, IE, vol. 265, 7 April 2018 (2018-04-07), pages 70 - 76, XP085402891, ISSN: 0165-1781, DOI: 10.1016/J.PSYCHRES.2018.03.080**
- **"Mood Disorders", 23 January 2013, INTECH, ISBN: 978-953-51-0959-4, article JANA HROUDOVA ET AL: "Mitochondrial Functions in Mood Disorders", XP055257147, DOI: 10.5772/53254**
- **OLDE LOOHUIS NIKKIE F M ET AL: "The schizophrenia risk geneMIR137acts as a hippocampal gene network node orchestrating the expression of genes relevant to nervous system development and function", PROGRESS IN NEURO-PSYCHOPHARMACOLOGY & BIOLOGICAL PSYCHIATRY, vol. 73, 27 February 2016 (2016-02-27), pages 109 - 118, XP029816963, ISSN: 0278-5846, DOI: 10.1016/J.PNPBP.2016.02.009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHEN JUNJUN ET AL: "Integrative analysis of mRNA and miRNA expression profiles in oral lichen planus: preliminary results", ORAL SURGERY, ORAL MEDICINE, ORAL PATHOLOGY AND ORAL RADIOLOGY, vol. 124, no. 4, 1 October 2017 (2017-10-01), pages 390, XP085233369, ISSN: 2212-4403, DOI: 10.1016/ J.OOOO.2017.05.513**
- **KENSUKE SAKAMOTO ET AL: "A comprehensive review of the genetic and biological evidence supports a role for MicroRNA-137 in the etiology of schizophrenia", AMERICAN JOURNAL OF MEDICAL GENETICS PART B: NEUROPSYCHIATRIC GENETICS, vol. 177, no. 2, 14 June 2017 (2017-06-14), pages 242 - 256, XP055696889, ISSN: 1552-4841, DOI: 10.1002/ajmg.b.32554**
- **M J GREEN ET AL: "Genome-wide supported variant MIR137 and severe negative symptoms predict membership of an impaired cognitive subtype of schizophrenia", MOLECULAR PSYCHIATRY, vol. 18, no. 7, 26 June 2012 (2012-06-26), GB, pages 774 - 780, XP055696893, ISSN: 1359-4184, DOI: 10.1038/mp.2012.84**
- **YAGAMI TATSUROU ET AL: "Pathophysiological Roles of Cyclooxygenases and Prostaglandins in the Central Nervous System", MOLECULAR NEUROBIOLOGY, HUMANA PRESS, US, vol. 53, no. 7, 2 September 2015 (2015-09-02), pages 4754 - 4771, XP036017859, ISSN: 0893-7648, [retrieved on 20150902], DOI: 10.1007/S12035-015-9355-3**
- **MA JIE ET AL: "Identification of miR-22-3p, miR-92a-3p, and miR-137 in peripheral blood as biomarker for schizophrenia", PSYCHIATRY RESEARCH, ELSEVIER IRELAND LTD, IE, vol. 265, 7 April 2018 (2018-04-07), pages 70 - 76, XP085402891, ISSN: 0165-1781, DOI: 10.1016/ J.PSYCHRES.2018.03.080**
- **"Mood Disorders", 23 January 2013, INTECH, ISBN: 978-953-51-0959-4, article JANA HROUDOVA ET AL: "Mitochondrial Functions in Mood Disorders", XP055257147, DOI: 10.5772/ 53254**
- **OLDE LOOHUIS NIKKIE F M ET AL: "The schizophrenia risk geneMIR137acts as a hippocampal gene network node orchestrating the expression of genes relevant to nervous system development and function", PROGRESS IN NEURO-PSYCHOPHARMACOLOGY & BIOLOGICAL PSYCHIATRY, vol. 73, 27 February 2016 (2016-02-27), pages 109 - 118, XP029816963, ISSN: 0278-5846, DOI: 10.1016/ J.PNPBP.2016.02.009**



- **CHEN JUNJUN ET AL: "Integrative analysis of mRNA and miRNA expression profiles in oral lichen planus: preliminary results", ORAL SURGERY, ORAL MEDICINE, ORAL PATHOLOGY AND ORAL RADIOLOGY, vol. 124, no. 4, 1 October 2017 (2017-10-01), pages 390, XP085233369, ISSN: 2212-4403, DOI: 10.1016/ J.OOOO.2017.05.513**
- **KENSUKE SAKAMOTO ET AL: "A comprehensive review of the genetic and biological evidence supports a role for MicroRNA-137 in the etiology of schizophrenia", AMERICAN JOURNAL OF MEDICAL GENETICS PART B: NEUROPSYCHIATRIC GENETICS, vol. 177, no. 2, 14 June 2017 (2017-06-14), pages 242 - 256, XP055696889, ISSN: 1552-4841, DOI: 10.1002/ajmg.b.32554**
- **M J GREEN ET AL: "Genome-wide supported variant MIR137 and severe negative symptoms predict membership of an impaired cognitive subtype of schizophrenia", MOLECULAR PSYCHIATRY, vol. 18, no. 7, 26 June 2012 (2012-06-26), GB, pages 774 - 780, XP055696893, ISSN: 1359-4184, DOI: 10.1038/mp.2012.84**
- **YAGAMI TATSUROU ET AL: "Pathophysiological Roles of Cyclooxygenases and Prostaglandins in the Central Nervous System", MOLECULAR NEUROBIOLOGY, HUMANA PRESS, US, vol. 53, no. 7, 2 September 2015 (2015-09-02), pages 4754 - 4771, XP036017859, ISSN: 0893-7648, [retrieved on 20150902], DOI: 10.1007/S12035-015-9355-3**


Remarks:

The complete document including Reference Table(s) and the Sequence Listing(s) can be downloaded from the EPO website

## Description

The present invention relates to the field of diagnostic and/or prognostic and/or subject stratification biomarker assays for the prognosis and/or diagnosis and/or therapy of high risk schizophrenia. The present invention also relates to the mitochondria-targeted antioxidant for use in the treatment of subjects classified as high-risk early psychosis subjects.

## Introduction

Schizophrenia is a debilitating psychiatric disorder characterized by asynchronous neural activity stemming from functional deficit in parvalbumin interneurons (PVIs). From the symptomatic point of view, the psychiatric diagnosis describes schizophrenia as a chronic, severe mental disorder, characterized by abnormalities in the perception or expression of reality. These include positive symptoms (hallucinations and delusions), negative symptoms (e.g. social withdrawal, blunted affect) and pervasive cognitive deficits that have been associated with social decline. Onset of the symptoms typically occurs in young adulthood, with approximately 0.4 - 0.6% of the population affected. Genetic factors have been shown as the main causes of schizophrenia, and the heritability of the disease has been shown to be at the level of 80%. As the full picture of the etiology of the disease as well as understanding of its heterogeneity remains limited, currently available pharmacological treatments have limited efficacy and continue to have adverse effects. Of note, schizophrenia is a representative example of a psychosis, a dimension that is present in several different psychiatric disorders. Its prevalence in the population can be as high as 5%.

Mitochondrial impairments could represent a key mechanism in the pathophysiological processes behind psychotic disorders, in particular behind schizophrenia. In recent years, oxidative stress has emerged as a core pathological feature in schizophrenia based on converging evidence from environmental and genetic studies linking this phenomenon to impairment of parvalbumin interneurons and subsequent cognitive control-related gamma oscillation deficits. Evidence suggests mitochondrial dysfunction in schizophrenia as indicated by loss of electron transport chain activity and a shift toward anaerobic energy metabolism.

Several studies provide evidence that the etiology of schizophrenia can be in part explained by oxidative stress. Oxidative stress is a state caused by an imbalance between the production of reactive oxygen species (ROS) and antioxidants that reduce ROS, leading to an accumulation of ROS. Evidence from environmental and genetic studies links this process to parvalbumin interneurons impairment. Disturbances in endogenous antioxidants, particularly glutathione (GSH), are observed, with decreased levels of this tripeptide reported in peripheral blood and prefrontal cortex of schizophrenia patients. Other evidence linking oxidative stress to schizophrenia refers to experiments and observations with transgenic mice carrying a permanent deficit of GSH synthesis (referred to as *Gclm*-KO). These mice exhibit widespread cortical deficiencies of parvalbumin interneurons and alterations of beta/gamma oscillations. Furthermore, when these mice that are exposed to GBR12909, a specific dopamine reuptake inhibitor that is useful to mimic the increased dopamine signaling observed in schizophrenia, the elevated levels of the oxidative DNA damage marker 8-oxo-7,8-dihydro-2'-deoxyguanosine is observed in the extranuclear compartment. It is indicative of mitochondrial DNA oxidation. These animals also exhibit increased levels of globular and dark mitochondria in neuropiles of parvalbumin interneurons, which is further consistent with inefficient clearance of damaged mitochondria through mitophagy.

Currently, diagnosis and stratification of patients into low and high risk patients requires a thorough psychological evaluation and a comprehensive medical exam to rule out other conditions, whereby the clinical high risk period is a phase denoting a risk for overt psychosis. However, no single symptom is characteristic of the disease and/or of the disease state. The classification as high-risk psychotic disorder subject, for example schizophrenia, involves individual evaluation of the severity of the characteristic symptoms and is based on the patient's self-reported experiences and observed behavior. The schizophrenia subjects are believed to be heterogeneous. The pathophysiology of schizophrenia remains unclear and there are no laboratory tests or biomarkers used as direct diagnostic and/or prognostic and/or stratification tool at present. Thus, there is a clear need to develop a laboratory-based test for classification of psychotic disorder subjects, which would be independent of often subjective clinical criteria. Indeed, mechanism-based molecular biomarkers, preferably biomarkers that can be obtained from peripheral blood samples, which would be evident for specific behavioral and cognitive alterations, are needed for classification of schizophrenia subjects, in view of treatments and prevention

Insofar as the present disclosure relates to psychotic disorders different from schizophrenia, the disclosure falls outside the claimed invention, but is kept in the description for illustrative purposes. MA JIE ET AL: "Identification of miR-22-3p, miR-92a-3p, and miR-137 in peripheral blood as biomarker for schizophrenia", PSYCHIATRY RESEARCH, ELSEVIER IRELAND LTD, IE, vol. 265, (2018), pages 70-76, ISSN: 0165-1781, DOI: 10.1016/J.PSYCHRES.2018.03.080 discloses that miR-137 can be used as biomarker for schizophrenia.

The underlying technical problem is thus the provision of methods for the classification of subjects having a psychotic disorder.

The technical problem is solved by embodiments provided herein and as characterized in the claims.

Thus, in the first aspect, this invention relates to a method for classifying a subject as a high-risk psychotic

disorder subject, the method comprising the steps of: (a) providing a biological sample obtained from a subject; (b) determining the expression level of miRNA-137, and the expression level of COX6A2; and (c) classifying the subject as high-risk psychotic disorder subject based on expression levels determined in step (b).

miR-137 is preferably defined herein as non-coding RNA molecule, with sequence according to SEQ ID NO: 1. (SEQ ID NO:1; UUAUUGCUUAAGAAUACGCGUAG). The sequence according to SEQ ID NO: 1 may also be referred to as a mature sequence of miR-137. As further defined herein, miR-137 is preferably defined herein as non-coding RNA molecule, wherein the microRNA (miRNA) stem loop comprises the sequence that is identical with sequence according to SEQ ID NO: 2. (SEQ ID NO: 2;

GGUCCUCUGACUCUCUUCGGUGACGGGUAUUCUUGGGUGGAUAAUACGGAUUA
CGUUGUUAUUGCUUAAGAAUACGCGUAGUCGAGGAGAGUACCAGCGGCA).

COX6A2 is preferably defined as a polypeptide that is identical with sequence according to SEQ ID NO: 3. (SEQ ID NO: 3; MALPLRPLTR GLASAAKGGH GGAGARTWRL LTFVLALPSV ALCTFNSYLH SGHRPRPEFR PYQHLRIRTK PYPWGDGNHT LFHNSHVNPL PTGYEHP).

Fig. 1 represents the correlation between the expression level of miR-137 and expression level of COX6A2 in differently classified groups of patients. It was unexpectedly found that there is significant correlation between the expression level of miR-137 and expression level of COX6A2 observed only in the group of subjects diagnosed as early psychosis subjects (as shown in the Examples). No such correlation is observed in the group classified as low risk psychotic disorder subjects. The combined observation of the expression levels of miR-137 and COX6A2 thus surprisingly leads to the classification of a subject as high-risk psychotic disorder subject.

One problem addressed by this invention is avoiding classifying subjects that are not high-risk schizophrenic subjects as high-risk schizophrenic subjects. In other words, it is important to avoid classifying healthy subjects or low-risk schizophrenic subjects as high-risk schizophrenic subjects. As it is further shown in the examples (Example 8, Figure 6), by classifying the subjects based on determining the concentrations of both biomarkers together it is possible to exclude low-risk schizophrenic subjects. This finding is unexpected to the person skilled in the art.

The use of miR-137 alone or its combination with other biomarkers disclosed herein does not allow for such accurate classification of subjects. Fig. 2 shows an example study of the performance of the use of the miR-137 biomarker alone and in combination with COX6A2, NIX, LC3B, FUNDC1, MMP9 and sRAGE, presented as the Receiver Operating Characteristic (ROC) curves. It is known to that skilled in the art that ROC curves are useful in assessing, if particular selection criteria can be useful in determining true positives and distinguishing them from true negatives. In this case, high-risk schizophrenic subjects are differentiated from low-risk schizophrenic subjects. It is known to that skilled in the art that area under curve parameter (AUC) is useful in comparing the performances of different selection criteria presented by ROC curves. It is unexpected to that skilled in the art that combination of miR-137 and COX6A2 performs significantly better that the other combinations, or the use of miR-137 biomarker alone.

In certain embodiments of this invention, for determining the patient status, the requirements set forth herein for both biomarkers are to be met. The requirements to be met by the biomarker refer to the values describing its expression level, obtained in the course of determination of their expression levels as disclosed herein, which are to be significantly altered from a certain threshold level. If only one of the biomarkers fulfills said requirements, or if none of the biomarkers fulfills the said requirements, the subject is not classified as high risk psychotic disorder subject.

The term high-risk psychotic disorder subject refers to a subject, patient or individual that is likely to develop schizophrenia in the future. The high risk psychotic disorder subject may be showing limited spectrum of symptoms of the psychotic disorder, including cognitive impairment, that is not all of the symptoms required for a complete diagnosis, and is likely to develop to full disease state in the future. The high-risk psychotic disorder subject may also have been diagnosed with early psychotic disorder.

The term "psychotic disorder" or "psychosis" refers to disease selected from the group containing schizophrenia, epilepsy, mood disorder, bipolar disorder (manic psychosis), cognitive impairment, age-associated disorders. Only schizophrenia falls within the scope of the claims. The term psychotic disorder is not limited to these examples and may also include other psychiatric disorders. The psychotic disorder may refer to early psychotic disorder, preferably it may refer to early schizophrenia.

The term "subject," as used herein refers to an individual organism, preferably a human that is affected by particular disorder. In some embodiments of this invention subjects may also be referred to as patients or as individuals. Therefore, the terms psychotic disorder subjects, psychotic disorder subjects and psychotic disorder individuals are herein used interchangeably.

As used herein, a term "biological sample" refers to a sample of biological material obtained from a subject, preferably a human subject, including but not limiting to a tissue, a tissue sample, cell sample, fluid sample. Non-limiting examples of such biological samples are serum, plasma, whole blood, blood-derived exosomes, plasma-derived

exosomes, neural exosomes, cerebrospinal fluid, cortical tissue, post-mortem brain tissue, urine. The biological sample can be subjected to a variety of post-collection procedures prior to assessing the amount of marker in the sample. These procedures may include, but are not limited to, nucleic acid and/or protein extraction, fixation, storage, freezing, ultrafiltration, concentration, evaporation, centrifugation. The purpose of subjecting the sample to these procedures is to prepare it for assessing the amount or level of biomarker in the sample. Furthermore, these procedures may allow for long-term storage of the sample, without affecting the biomarker amount or level.

The term "expression level" of a biomarker, is a detectable level in a biological sample. It can be measured by methods known to one skilled in the art and are also disclosed herein. The expression level of biomarkers assessed can be used to stratify groups of patients. The term "expression" generally refers to the process by which information (e.g. gene-encoded and/or epigenetic) is converted into the biological molecules of a different type or function, that are present and operate in the cells. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g. posttranslational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (e.g. microRNAs).

The term "significantly higher level of expression" of a marker refers to an expression level in a test sample that is greater than the standard error of the assays employed to asses expression, and in certain embodiments, at least twice, and in other embodiments, three, four, five or ten times the expression level of the marker compared to a specified threshold expression level or concentration.

The term "significantly lower level of expression" of a marker refers to an expression level in a test sample that is lower than the standard error of the assays employed to asses expression, and in certain embodiments, at least twice, and in other embodiments, three, four, five or ten times the expression level of the marker compared to a specified threshold expression level or concentration.

The term "about", when considering value X obtained in the course of the measurement, and used in the context "about X" refers to the value similar to X, preferably with 10% of value X, more preferably within 5 % of value X or within 2 % of value X. For example, the term "about 10 ng/mL" would mean between 9 and 11 ng/mL, or more preferably a value between 9.5 and 10.5 ng/mL, or between 9.8 and 10.2 ng/mL.

Unless defined otherwise, all technical and scientific terms used herein have the same meanings as commonly understood by one of ordinary skill in the art to which this invention belongs.

The term "biomarker", as used herein refers to an indicator, e.g. predictive, diagnostic, and/ or prognostic, which can be detected in a sample. The biomarker may serve as an indicator of a particular subtype of a disease or disorder, most preferably schizophrenia, characterized by certain molecular, pathological, histological, and/ or clinical features. In the most preferred embodiment the biomarker is a gene, polynucleotide (e.g. DNA and/or RNA) and/or protein and/ or functional variants thereof, whose altered level of expression in a sample is associated with a disorder and/or disease state.

The term RNA refers to a polyribonucleotide of any conceivable length (it may be less than ten nucleotides, it may be more than 100 nucleotides, or more than 1000 nucleotides), it may also refer to a covalently modified polyribonucleotide, of molecular structures comprising at least two polyribonucleotides that are engaged in base pairing. The term ribonucleotide refer to any of the four naturally occurring ribonucleotides, including adenosine, guanosine, uridine and cytidine, the term modified ribonucleotide may refer to covalent modifications thereof. Messenger RNA or mRNA refers to RNA carrying genetic information that will be translated into protein products at the ribosomes.

The measurements of the expression levels of polynucleotide biomarkers are useful in diagnosis, classification or stratification of subjects as psychotic disorder subjects, preferably early schizophrenia subjects. Polynucleotide may refer to oligoribonucleotide, and more preferably biomarker may refer to micro RNA (also referred to as miRNA or as miR-XXX, wherein XXX stands for number of a code that identifies specific micro RNA). A miRNA comprises an antisense. However, this is not a limiting feature and in some cases micro RNA may also comprise of a sense strand. A miRNA antisense strand is generally 18-25 nt long and binds to fully or partially complementary messenger RNAs, thereby targeting them for degradation or translational inhibition. Many micro RNA molecules naturally participate in gene regulation; they are indigenously produced in the cells of many organisms, including humans. The significantly higher or significantly lower level of expression of miRNAs may promote disease state. Therefore, the significantly higher or significantly lower level of expression of miRNAs may also be indicative of a disease stage, and may be used as biomarker in diagnosis of the disease state or in stratification and classification of subjects and/or patients. As up to 70% of the human miRNAs are expressed in nervous system, miRNAs are useful as biomarkers of psychotic disorders. For a non-limiting example of psychotic disorder, miRNA biomarkers are useful as biomarkers of schizophrenia. In the case of schizophrenia, the following miRNAs have been proposed as potential biomarkers: miR-30e, mi-R130b, mi-R193a-p3, mi-R30e,

miR181b, miR-181b, miR-34a, miR-346, miR-7, miR-449a, miR-564, miR-432, miR-548d, miR-572, miR-652, miR-652, mi-R30a, miR-137.

As disclosed in this invention, the micro RNA 137, referred to as miR-137, can be used as a biomarker in diagnosis, classification or stratification of subjects as psychotic disorder subjects, preferably early schizophrenia subjects, as outlined above. Quantitative analysis of plasma composition has revealed that exosomal miR-137 levels are significantly higher in the early psychosis patient group compared with age- and gender-matched healthy controls, as evidenced using the samples obtained from human subjects. To demonstrate that, eligible patients were recruited from the Treatment and Early Intervention in Psychosis Program (TIPP, Lausanne University Hospital, Switzerland, which is a specialized 3-year program for the treatment of early psychosis patients. The early psychosis patients, as it is outlined below in the examples, are subjects determined as such following the clinical evaluation. Inclusion criteria were: (i) individuals aged 18 to 35 years old; (ii) residence in Lausanne and surroundings areas; (iii) meeting threshold criteria for psychosis, as defined by the 'Psychosis threshold' subscale of the Comprehensive Assessment of At Risk Mental State; (iv) no more than 6 months of treatment with antipsychotic medication for psychosis; (v) no psychosis related to intoxication or organic brain disease; (vi) intelligence quotient ≥ 70; and (vii) ability to discern and to provide informed consent. The psychosis threshold and the diagnosis assessment resulted from an expert consensus including a senior psychiatrist, a psychologist and the case manager who closely followed-up the patient during the 3-year program. The duration of the illness considered the time elapsed from the psychosis threshold to the participation in the study and the diagnosis was based on Diagnostic and Statistical Manual of Mental Disorders criteria, which are well known to that skilled in the art. Healthy controls were recruited within same catchment area. They were in order to attest for the absence of any major psychiatric or substance use disorder. In addition, healthy controls who reported having a first-degree relative with psychotic disorder were excluded. Neurological disorders and severe head trauma were also exclusion criteria for all subjects.

The gene encoding for miR-137 has been shown to bear a polymorphism, which has been reported as a potential regulator for schizophrenia susceptibility, as well as genetic marker thereof. Compared to the low risk genotype miR-137 early psychosis patient group and control group, the high risk miR-137 early psychosis patient genotype group exhibited significantly higher levels of plasma miR-137. Of note, increased miR-137 level correlated with the observed clinical parameters. As outlined in the examples, deficits in intertrial coherence (ITC) and evoked power (ePOW) in Auditory Steady State Responses (ASSR) were restricted to high risk miR-137 early psychosis patient genotype group and were not observed in low risk miR-137 early psychosis patient genotype group or either of the control groups. These findings show that subjects in the early psychotic disorder subject group harboring the high risk genotype clearly differentiate from those without the mutation that predisposes them for psychotic disorder. High risk psychotic disorder subjects exhibit more significant positive symptoms, lower functional outcome (GAF) and more severe cognitive impairment, which is measured by assessing processing speed, attention /vigilance, working memory and verbal learning.

In certain embodiments of this invention, protein biomarkers can also be used in the process of classifying, stratifying or diagnosing subjects with psychiatric disorder. It has been shown that psychotic disorders are linked to mitochondrial disorders. Defective mitochondria, that means mitochondria that have suffered in the course of damage or stress conditions, undergo the process of their selective degradation through autophagy. Such selective degradation of mitochondria is referred to as mitophagy. Connection between mitophagy and psychotic disorders has been demonstrated by measuring the levels of mitophagy biomarkers in the samples obtained from early psychotic disorder patients. To this end, plasma levels of the mitophagy receptors NIX and FUNDC1 as well as the autophagosome LC3B were lower in the early psychosis subject group compared to subjects from the control group, suggestive of a mitophagy defect in these individuals. Furthermore, these markers of mitophagy including NIX, FUNDC1 and LC3B are significantly decreased in the animal models of the disorder, which are discussed in examples in more details. Thus, the oxidative-stress-induced upregulation of miR-137 leading to mitophagy markers deficit observed centrally in animal model could be reflected peripherally in early psychosis patients so that high expression level of miR-137 observed in plasma can plausibly be a marker representative of parvalbumin interneuron impairments. Molecular biomarkers that are related to mitophagy processes could also be useful in classifying subjects as high-risk psychotic disorder subject. The example biomarkers that can be useful for this purpose include, but are not limited to, NIX, FUNDC1, LC3B.

COX6A2 is another important mitochondrial marker. It stands for cytochrome c oxidase subunit VIa polypeptide 2, and it is a subunit of cytochrome c oxidase (COX), a multisubunit enzyme complex that couples the transfer of electrons from cytochrome c to molecular oxygen, and plays a role in creating a proton electrochemical gradient across the inner mitochondrial membrane, which in turns contributes to the ATP synthesis. COX is a terminal enzyme of the mitochondrial respiratory chain. Importantly, COX6A2 co-localizes in brain only with parvalbumin-positive cells. It has been unexpectedly found that decreased level of COX6A2, but not of some other subunits of the COX complex, is seen only for high risk early psychotic disorder subjects and not for the low risk subjects.

The measurements of the biomarker level are to be performed with sufficient confidence, as it is understood by the one skilled in the art, to determine beyond any doubt that the subject can be classified as a high risk psychotic disorder subject or as a non-high risk psychotic disorder subject. Should the measurements of any one of the biomarkers provide a

value about the threshold, as set forth in this application, it is known to that skilled in the art that these measurements can be repeated until suitable level of confidence is reached.

The method of the invention relates to an embodiment, wherein the psychotic disorder is schizophrenia;

The term psychotic disorder is not limited to these examples and may also include other psychiatric disorders. The psychotic disorder may refer to early psychotic disorder, preferably it may refer to early schizophrenia, early epilepsy, early bipolar disorder. The classification of disorders disclosed herein is done according to mostly clinical and behavioral criteria known to the one skilled in the art. This invention provides the means of classification of subjects that are based on measurements of molecular biomarkers. The methods described herein can be used to complement clinical diagnosis and classification methods. Furthermore, as the methods described here are less laborious and more cost-effective, they can also be used to replace clinical diagnosis and classification methods and screen and classify larger population of subjects. As molecular biomarkers rely on clear criteria for classification of subjects, it is also expected that classification will be less subjective and more accurate.

A further preferred aspect of the method of this invention relates to an embodiment, wherein the classification comprises differential classification between high-risk psychotic disorder subjects and low-risk psychotic disorder subjects. The term high-risk psychotic disorder subject refers to a subject, patient or individual that is likely to develop a psychotic disorder in the future. The high risk psychotic disorder subject may be showing some symptoms of the psychotic disorder and is likely to develop to full disease state in the future. The high-risk psychotic disorder subject may also have been diagnosed with early psychotic disorder. The term differential classification may refer to taking a decision into which of two or more groups a subject should be classified. The decision is taken based on the measurements of two biomarkers, the expression level of miR-137 and the protein level of COX6A2, as disclosed herein.

Another particular aspect of the method of this invention relates to an embodiment, wherein the biological sample comprises peripheral blood, plasma, serum, cerebrospinal fluid, blood-derived exosomes, plasma-derived exosomes, neural exosomes, cortical tissue, post-mortem brain tissue, fibroblast cell culture, induced pluripotent stem cell culture, derived neuronal precursor cell culture. In certain embodiments of this invention the level of expression of the marker can be assessed by assessing the amount (e.g. absolute amount or concentration) of the marker in a biological sample. The biological sample can be subjected to a variety of post-collection procedures prior to assessing the amount of marker in the sample. These procedures may include, but are not limited to, nucleic acid and/or protein extraction, fixation, storage, freezing, ultrafiltration, concentration, evaporation, centrifugation. The purpose of subjecting the sample to these procedures is to prepare it for assessing the amount or level of biomarker in the sample. Furthermore, these procedures may allow for long-term storage of the sample, without affecting the biomarker amount or level. In certain embodiments of this invention the level of biomarkers can be measured by using different measurement approaches. For example, the level of biomarkers can be measured in a biological sample obtained from a subject. Non-limiting examples of such biological samples that are useful for biomarker determination are serum, plasma, whole blood, blood-derived exosomes, plasma-derived exosomes, neural exosomes, cerebrospinal fluid, cortical tissue, post-mortem brain tissue, urine. In a preferred embodiment of this invention, the level of particular biomarker can be measured in the sample containing exosomes derived from human body fluids (plasma, serum and urine).

In certain embodiments of this invention, both biomarkers can be measured in the same biological sample. However, this feature is not limiting and in certain embodiments of this invention different biomarkers can be measured in different samples coming from the same subject. The samples can be collected at the same time, but they can also be collected on different times at the same day, or on different days. In the preferred embodiment of this invention, both biomarkers are determined using material coming from the same biological sample.

In this invention expression level of miR-137 and expression level of COX6A2 are determined by an *in vitro* assay. Quantification of the biomarkers used herein may be accomplished by any of established *in vitro* techniques that are useful for this purpose, known to that skilled in the art. These techniques may include, but not limited to, immunoassay; an ELISA-based assay, an aptamer-based assay; an mRNA expression level assay; *in situ* hybridization assay; a PCR-based assay; a real time PCR-based assay, next generation sequencing; an electrochemistry-based assay; a lateral-flow assay; a nanobead-based assay; a microfluidics-based assay; and an oligonucleotide-templated reaction.

It is known to that skilled in the art that several different *in vitro* assays are useful in determination of cellular levels of RNA molecules. In particular, *in vitro* assay is selected from the group consisting of an immunoassay; an ELISA-based assay, an aptamer-based assay; an mRNA expression level assay; *in situ* hybridization assay; a proteomics-based assay; a PCR-based assay; a real time PCR-based assay, next generation sequencing; an electrochemistry-based assay; a lateral-flow assay; a nanobead-based assay; a microfluidics-based assay; and an oligonucleotide-templated reaction. The techniques useful for this purpose are not limited to these examples.

In a preferred embodiment of this invention, the level of microRNA can be measured by using the RT PCR-based assay. As it would be known to that skilled in the art, quantification of gene expression using RT PCR comprises measuring relative gene expression level, as compared to the reference genes, that is, the genes that maintain constant expression in different subjects, conditions, disease state. Reference genes may be selected for example by using global quantitative transcriptomic analysis approaches, including but not limited to next generation sequencing. As known to that skilled in the

art, reference genes may include, but are not limited to, miR-16, snRNA-U1 and snRNA-U6. In the embodiment of this invention relating to quantification of miRNAs, the house keeping genes may comprise other miRNAs, for example miR-16. In another embodiment of this invention, reference genes may include RNA particles that are comprised within functional complexes of RNA and proteins, in particular they may include RNA particles comprised in splicosomes or ribosomes. Preferably, such reference genes may include, but are not limited to, snRNA-U1 and snRNA-U6. In another particular embodiment of this invention an RNA species of non-sample origin can be introduced to the sample in known amount for the purposes of quantification of other selected RNA species. This approach, known to that skilled in the art as spike-in, may include (but is not limited to) the use of miR-39 *C. elegans* at $1.6 \times 10^8$ copies/μl. In the most preferred embodiment of this invention, a group of three reference genes, comprising miR-16, snRNA-U1 and snRNA-U6, is used. As the result of the measurement is given relative to the expression level of the reference genes, it will be expressed in as the fold change compared to the control, or the arbitrary units (a.u.). Arbitrary units make reference to relative level of miR-137 expression, relative to the average expression level of the reference genes used therein.

RT PCR methodology, as known to that skilled in the art, comprises reverse transcription step wherein from an RNA transcript a corresponding DNA product is produced, and a polymerase chain reaction (PCR) step, wherein the said DNA product is amplified, that means, copies of this DNA are produced by using polymerase useful for this purpose, short oligodeoxyribonucleotides that are complimentary to the DNA product in such a way that can serve as a starting point of PCR reaction, and deoxyribonucleotides provided in the buffer. It is known to that skilled in the art that amount of particular RNA in the sample is quantified by using the term of cycle threshold or Ct. In the polymerase chain reaction, the transcripts are amplified in each cycle of PCR reaction and under optimal conditions the concentration of the PCR product will double with each cycle. RT PCR experiment can be performed in such a way, that amount of PCR product is monitored in real time by the use of a fluorescent probe and spectrophotometer useful for this purpose (also known to those skilled in the art as the light cycler). Dependent on the amount of template RNA in the sample (and so amount of corresponding DNA upon reverse transcription), the PCR product can be detected in the sample after a number of cycles - this number is referred to as cycle threshold or Ct. It is known to that skilled in the art that such quantification method is very sensitive to variations of total sample amount, which can be introduced by pipetting errors, among others. Therefore, RT PCR experiments are always performed for several transcripts, and quantification is performed relatively to a reference gene. It is expressed as difference between cycle threshold for both transcripts, delta Ct, and it depends only on the ratio of both transcripts and is independent of the total sample amount. When comparing deltaCt parameters for different conditions, for examples for samples coming from different patients, the difference between delta Ct parameters is used. The delta delta Ct parameter allows for calculation of the fold change of selected transcript, and can be expressed according to the formula:

$$\text{Fold change} = 2 \wedge (\text{-delta delta Ct})$$

In a preferred embodiment of this invention, miRNA biomarker is quantified in the biological sample comprising exosomes isolated from human plasma. Exosomes are extracellular vesicles that are permeable to the blood brain barrier. Therefore, exosomes that originate from the brain cells can be accessible through peripheral fluids such as plasma, serum, blood or urine. The origin of the exosomes can be assessed by determining the presence and level of the neural markers in the samples containing the exosomes. It is known to that skilled in the art that the exosomes can be isolated from blood by the process of ultracentrifugation.

A particular aspect of the method of this invention relates to an embodiment, wherein the relative expression level of miR-137 normalised to reference genes is higher than a threshold value. In some embodiments of this invention, subjects are classified as psychosis subjects if the expression level of miR-137 is significantly altered from a certain threshold. The term "significantly altered level of expression" of a marker refers to an expression level in a test sample that is greater than the standard error of the assays employed to asses expression, and in certain embodiments, at least twice, and in other embodiments, three, four, five or ten times the expression level of the marker compared to a specified threshold expression level or concentration. The threshold may be set with respect to the average miR-137 expression level of healthy subject population. In certain embodiments of this invention, the significantly altered level of expression may refer to a significantly higher level of expression mi miR-137. The significantly higher level of expression of miR-137 may preferably refer to at least 1.25-fold increase, 1.5-fold increase, 2-fold increase, 2.5 fold increase, 5 fold increase, 7.5 fold increase, or 10-fold increase in the expression level of miR-137 compared to the control. In a more preferred embodiment of this invention, the threshold may refer to 1.5 fold increase, 2-fold increase, 2.5 fold increase or 5-fold increase in the expression of miR-137 compared to the control. In the most preferred embodiment of this invention, the threshold may be set at at least 2-fold increase of miR-137 expression level compared to the control.

The threshold may also be expressed in the arbitrary units, with respect to the expression level of reference genes. Using these units, the significantly higher level of expression of miR-137 would correspond to the expression level higher than 3.0 a.u, higher than, 3.6 a.u., higher than 4.8 a.u., higher than 6.0 a.u., higher than 12 a.u., higher than 18 a.u., or higher than 24 a.u.. In a more preferred embodiment of this invention, the threshold may refer to the expression level

higher than 3.6 a.u., higher than 4.8 a.u., higher than 6.0 a.u., or higher than 12 a.u.. In the most preferred embodiment of this invention, the threshold may be set as higher than 4.8 a.u..

A preferred aspect of the method of the invention relates to an embodiment, wherein the threshold value of miR-137 expression level is 4.8 a.u., as normalized to the average expression level of miR-16, snRNA-U1 and snRNA-U6. This threshold corresponds to set at 2-fold increase of miR-137 expression level. In the most preferred embodiment of this invention, a group of three reference genes, comprising miR-16, snRNA-U1 and snRNA-U6, is used. Thus a preferred aspect of the method of the invention relates to an embodiment, wherein the threshold value of miR-137 expression level corresponds to a 2-fold increase over the average expression level of healthy subject.

Fig 3. shows the plot of miR-137 expression level in the different groups of subjects. Expression levels are significantly higher than in the other groups. However, several subjects that belong to other groups, i.e. low risk early psychotic disorder subjects or control healthy subjects, exhibit value of miR-137 expression level satisfying the threshold criteria. miR-137 biomarker is therefore not sufficient for classification of the patients as high-risk early psychotic disorder subjects, as described herein.

Further preferred aspect of the method of this invention relates to an embodiment, wherein the expression level of COX6A2 is lower than a threshold value.

COX6A2 is a protein, and it is known to that skilled in the art that the quantification of the protein level can be done by s variety of established *in vitro* techniques. Examples of techniques useful for this purpose include, but are not limited to, immunoassay; an ELISA-based assay, an aptamer-based assay, a proteomics-based assay, an electrochemistry-based assay; a lateral-flow assay; a nanobead-based assay; a microfluidics-based assay, a colorimetric assay, a fluorimetric assay, Western blotting. Any of these techniques can be used to measure the expression level of COX6A2. COX6A2 is associated with mitophagy, and similar to other mitophagy markers its level is lowered

In certain embodiments of this invention, Western Blot may be used to quantify the level of COX6A2. As it is known to a person skilled in the art, protein samples isolated originating from a biological sample obtained from the subject are separated on an SDS-PAGE gel and transferred to nitrocellulose membranes. Specific antibodies are used to detect the presence of COX6A2 in the sample. For example, anti-COX6A2 antibody is useful for detection of the presence of COX6A2 at the immunoblot. Presence of COX6A2 in the immunoblot is then revealed by using appropriate secondary antibody with suitable detection system. Detection systems are known to a person skilled in the art and may include horseradish peroxidase coupled to the secondary antibody and appropriate substrate that can be detected, for example colorimetrically or by measuring luminescence. For this purpose, a spectrophotometer of any design or make can be useful.

In further embodiments of this invention, ELISA assay can be used to detect and quantify the presence of COX6A2 in the sample. ELISA assay, as it is known to a person skilled in the art, comprises of contacting the sample with solid support to immobilize the sample, and detecting presence of the COX6A2 protein by using a specific antibody and a specific detection system. In a preferred embodiment, specific antibody and detection system are provided together as anti-COX6A2 antibody conjugated to horseradish peroxidase. The amount of protein of interest in the sample can be measured for example colorimetrically or by measuring luminescence. This is not considered to be a limiting feature.

In some embodiments of the invention the threshold for classifying a subject as high-risk psychosis subject may refer to below 2.0 ng/mL, to below 1.8 ng/mL, to below 1.6 ng/mL, to below 1.4 ng/mL, to below 1.2 ng/mL, to below 1.0 ng/mL, to below 0.8 ng/mL, to below 0.6 ng/mL, to below 0.4 ng/mL or to below 0.2 ng/mL In a preferred embodiment of this invention, the threshold is set as below 1.4 ng/mL, below 1.2 ng/mL, or below 1.0 ng/mL. In the most preferred embodiment of this invention, the threshold is set as below 1.2 ng/mL.

Therefore, one preferred aspect of the method of this invention relates to an embodiment, wherein the threshold value of COX6A2 expression level is 1.2 ng/ml.

In the embodiments disclosed herein, expression levels of two biomarkers are determined in the sample or samples that are provided from each subject. Subject can be classified as high-risk psychotic disorder subject only, when both biomarkers mR-137 and COX6A2 fulfil the requirements set forth herein. In some embodiments of this invention the subject is classified as high-risk early psychotic disorder subject based on level of expression of COX6A2 below 2.0 ng/mL and level of expression of miR-137 corresponding to at least 1.25-fold increase compared to the control, based on level of expression of COX6A2 below 2.0 ng/mL and level of expression of miR-137 corresponding to at least 1.5-fold increase compared to the control, based on level of expression of COX6A2 below 2.0 ng/mL and level of expression of miR-137 corresponding to at least 2-fold increase compared to the control, based on level of expression of COX6A2 below 2.0 ng/mL and level of expression of miR-137 corresponding to at least 2.5 fold increase compared to the control, based on level of expression of COX6A2 below 2.0 ng/mL and level of expression of miR-137 corresponding to at least 5 fold increase compared to the control, based on level of expression of COX6A2 below 2.0 ng/mL and level of expression of miR-137 corresponding to at least 7.5 fold increase compared to the control, based on level of expression of COX6A2 below 2.0 ng/mL and level of expression of miR-137 corresponding to or at least 10-fold increase compared to the control.

In some embodiments of this invention the subject is classified as high-risk early psychotic disorder subject based on level of expression of COX6A2 below 1.8 ng/mL and level of expression of miR-137 corresponding to 1.25-fold

increase compared to the control, based on level of expression of COX6A2 below 1.8 ng/mL and level of expression of miR-137 corresponding to at least 1.5-fold increase, based on level of expression of COX6A2 below 1.8 ng/mL and level of expression of miR-137 corresponding to at least 2-fold increase compared to the control, based on level of expression of COX6A2 below 1.8 ng/mL and level of expression of miR-137 corresponding to at least 2.5 fold increase compared to the control, based on level of expression of COX6A2 below 1.8 ng/mL and level of expression of miR-137 corresponding to at least 5 fold increase compared to the control, based on level of expression of COX6A2 below 1.8 ng/mL and level of expression of miR-137 corresponding to at least 7.5 fold increase compared to the control, based on level of expression of COX6A2 below 1.8 ng/mL and level of expression of miR-137 corresponding to at least 10-fold increase compared to the control.

In some embodiments of this invention the subject is classified as high-risk early psychotic disorder subject based on level of expression of COX6A2 below 1.6 ng/mL and level of expression of miR-137 corresponding to at least 1.25-fold increase compared to the control, based on level of expression of COX6A2 below 1.6 ng/mL and level of expression of miR-137 corresponding to at least 1.5-fold increase compared to the control, based on level of expression of COX6A2 below 1.6 ng/mL and level of expression of miR-137 corresponding to at least 2-fold increase compared to the control, based on level of expression of COX6A2 below 1.6 ng/mL and level of expression of miR-137 corresponding to at least 2.5 fold increase compared to the control, based on level of expression of COX6A2 below 1.6 ng/mL and level of expression of miR-137 corresponding to at least 5 fold increase compared to the control, based on level of expression of COX6A2 below 1.6 ng/mL and level of expression of miR-137 corresponding to at least 7.5 fold increase compared to the control, based on level of expression of COX6A2 below 1.6 ng/mL and level of expression of miR-137 corresponding to at least 10-fold increase compared to the control.

In some embodiments of this invention the subject is classified as high-risk early psychotic disorder subject based on level of expression of COX6A2 below 1.4 ng/mL and level of expression of miR-137 corresponding to at least 1.25-fold increase compared to the control, based on level of expression of COX6A2 below 1.4 ng/mL and level of expression of miR-137 corresponding to at least 1.5-fold increase compared to the control, based on level of expression of COX6A2 below 1.4 ng/mL and level of expression of miR-137 corresponding to at least 2-fold increase compared to the control, based on level of expression of COX6A2 below 1.4 ng/mL and level of expression of miR-137 corresponding to at least 2.5 fold increase compared to the control, based on level of expression of COX6A2 below 1.4 ng/mL and level of expression of miR-137 corresponding to at least 5 fold increase compared to the control, based on level of expression of COX6A2 below 1.4 ng/mL and level of expression of miR-137 corresponding to at least 7.5 fold increase compared to the control, based on level of expression of COX6A2 below 1.4 ng/mL and level of expression of miR-137 corresponding to at least 10-fold increase compared to the control.

In some embodiments of this invention the subject is classified as high-risk early psychotic disorder subject based on level of expression of COX6A2 below 1.2 ng/mL and level of expression of miR-137 corresponding to at least 1.25-fold increase compared to the control, based on level of expression of COX6A2 below 1.2 ng/mL and level of expression of miR-137 corresponding to at least 1.5-fold increase compared to the control, based on level of expression of COX6A2 below 1.2 ng/mL and level of expression of miR-137 corresponding to at least 2-fold increase compared to the control, based on level of expression of COX6A2 below 1.2 ng/mL and level of expression of miR-137 corresponding to at least 2.5 fold increase compared to the control, based on level of expression of COX6A2 below 1.2 ng/mL and level of expression of miR-137 corresponding to at least 5 fold increase compared to the control, based on level of expression of COX6A2 below 1.2 ng/mL and level of expression of miR-137 corresponding to at least 7.5 fold increase compared to the control, based on level of expression of COX6A2 below 1.2 ng/mL and level of expression of miR-137 corresponding to or at least 10-fold increase compared to the control.

In some embodiments of this invention the subject is classified as high-risk early psychotic disorder subject based on level of expression of COX6A2 below 1.0 ng/mL and level of expression of miR-137 corresponding to at least 1.25-fold increase compared to the control, based on level of expression of COX6A2 below 1.0 ng/mL and level of expression of miR-137 corresponding to at least 1.5-fold increase compared to the control, based on level of expression of COX6A2 below 1.0 ng/mL and level of expression of miR-137 corresponding to at least 2-fold increase compared to the control, based on level of expression of COX6A2 below 1.0 ng/mL and level of expression of miR-137 corresponding to at least 2.5 fold increase compared to the control, based on level of expression of COX6A2 below 1.0 ng/mL and level of expression of miR-137 corresponding to at least 5 fold increase compared to the control, based on level of expression of COX6A2 below 1.0 ng/mL and level of expression of miR-137 corresponding to at least 7.5 fold increase compared to the control, based on level of expression of COX6A2 below 1.0 ng/mL and level of expression of miR-137 corresponding to or at least 10-fold increase compared to the control.

In some embodiments of this invention the subject is classified as high-risk early psychotic disorder subject based on level of expression of COX6A2 below 0.8 ng/mL and level of expression of miR-137 corresponding to at least 1.25-fold increase compared to the control, based on level of expression of COX6A2 below 0.8 ng/mL and level of expression of miR-137 corresponding to at least 1.5-fold increase compared to the control, based on level of expression of COX6A2 below 0.8 ng/mL and level of expression of miR-137 corresponding to at least 2-fold increase compared to the

control, based on level of expression of COX6A2 below 0.8 ng/mL and level of expression of miR-137 corresponding to at least 2.5 fold increase compared to the control, based on level of expression of COX6A2 below 0.8 ng/mL and level of expression of miR-137 corresponding to at least 5 fold increase compared to the control, based on level of expression of COX6A2 below 0.8 ng/mL and level of expression of miR-137 corresponding to at least 7.5 fold increase compared to the control, based on level of expression of COX6A2 below 0.8 ng/mL and level of expression of miR-137 corresponding to or at least 10-fold increase compared to the control.

In some embodiments of this invention the subject is classified as high-risk early psychotic disorder subject based on level of expression of COX6A2 below 0.6 ng/mL and level of expression of miR-137 corresponding to at least 1.25-fold increase compared to the control, based on level of expression of COX6A2 below 0.6 ng/mL and level of expression of miR-137 corresponding to at least 1.5-fold increase compared to the control, based on level of expression of COX6A2 below 0.6 ng/mL and level of expression of miR-137 corresponding to at least 2-fold increase compared to the control, based on level of expression of COX6A2 below 0.6 ng/mL and level of expression of miR-137 corresponding to at least 2.5 fold increase compared to the control, based on level of expression of COX6A2 below 0.6 ng/mL and level of expression of miR-137 corresponding to at least 5 fold increase compared to the control, based on level of expression of COX6A2 below 0.6 ng/mL and level of expression of miR-137 corresponding to at least 7.5 fold increase compared to the control, based on level of expression of COX6A2 below 0.6 ng/mL and level of expression of miR-137 corresponding to or at least 10-fold increase compared to the control.

In some embodiments of this invention the subject is classified as high-risk early psychotic disorder subject based on level of expression of COX6A2 below 0.4 ng/mL and level of expression of miR-137 corresponding to at least 1.25-fold increase compared to the control, based on level of expression of COX6A2 below 0.4 ng/mL and level of expression of miR-137 corresponding to at least 1.5-fold increase compared to the control, based on level of expression of COX6A2 below 0.4 ng/mL and level of expression of miR-137 corresponding to at least 2-fold increase compared to the control, based on level of expression of COX6A2 below 0.4 ng/mL and level of expression of miR-137 corresponding to at least 2.5 fold increase compared to the control, based on level of expression of COX6A2 below 0.4 ng/mL and level of expression of miR-137 corresponding to at least 5 fold increase compared to the control, based on level of expression of COX6A2 below 0.4 ng/mL and level of expression of miR-137 corresponding to at least 7.5 fold increase compared to the control, based on level of expression of COX6A2 below 0.4 ng/mL and level of expression of miR-137 corresponding to or at least 10-fold increase compared to the control.

In some embodiments of this invention the subject is classified as high-risk early psychotic disorder subject based on level of expression of COX6A2 below 0.2 ng/mL and level of expression of miR-137 corresponding to at least 1.25-fold increase compared to the control, based on level of expression of COX6A2 below 0.2 ng/mL and level of expression of miR-137 corresponding to at least 1.5-fold increase compared to the control, based on level of expression of COX6A2 below 0.2 ng/mL and level of expression of miR-137 corresponding to at least 2-fold increase compared to the control, based on level of expression of COX6A2 below 0.2 ng/mL and level of expression of miR-137 corresponding to at least 2.5 fold increase compared to the control, based on level of expression of COX6A2 below 0.2 ng/mL and level of expression of miR-137 corresponding to at least 5 fold increase compared to the control, based on level of expression of COX6A2 below 0.2 ng/mL and level of expression of miR-137 corresponding to at least 7.5 fold increase compared to the control, based on level of expression of COX6A2 below 0.2 ng/mL and level of expression of miR-137 corresponding to at least 10-fold increase compared to the control.

In a more preferred embodiment of this invention, the subject is classified as high-risk early psychotic disorder subject based on expression level of COX6A2 below 1.4 ng/mL and level of expression of miR-137 corresponding to at least 1.25-fold increase compared to the control, based on expression level of COX6A2 below 1.4 ng/mL and level of expression of miR-137 corresponding to at least 1.5 fold increase compared to the control, based on expression level of COX6A2 below 1.4 ng/mL and level of expression of miR-137 corresponding to at least 2-fold increase corresponding to the control, based on expression level of COX6A2 below 1.4 ng/mL and level of expression of miR-137 corresponding to at least 2.5 fold increase corresponding to the control, or based on expression level of COX6A2 below 1.4 ng/mL and level of expression of miR-137 corresponding to at least 5-fold increase compared to the control.

In another more preferred embodiment of this invention, the subject is classified as high-risk early psychotic disorder subject based on expression level of COX6A2 below 1.2 ng/mL and level of expression of miR-137 corresponding to at least 1.25-fold increase compared to the control, based on expression level of COX6A2 below 1.2 ng/mL and level of expression of miR-137 corresponding to at least 1.5 fold increase compared to the control, based on expression level of COX6A2 below 1.2 ng/mL and level of expression of miR-137 corresponding to at least 2-fold increase corresponding to the control, based on expression level of COX6A2 below 1.2 ng/mL and level of expression of miR-137 corresponding to at least 2.5 fold increase corresponding to the control, or based on expression level of COX6A2 below 1.2 ng/mL and level of expression of miR-137 corresponding to at least 5-fold increase compared to the control.

In another more preferred embodiment of this invention, the subject is classified as high-risk early psychotic disorder subject based on expression level of COX6A2 below 1.0 ng/mL and level of expression of miR-137 corresponding to at least 1.25-fold increase compared to the control, based on expression level of COX6A2 below 1.0 ng/mL and level of

expression of miR-137 corresponding to at least 1.5 fold increase compared to the control, based on expression level of COX6A2 below 1.0 ng/mL and level of expression of miR-137 corresponding to at least 2-fold increase corresponding to the control, based on expression level of COX6A2 below 1.0 ng/mL and level of expression of miR-137 corresponding to at least 2.5 fold increase corresponding to the control, or based on expression level of COX6A2 below 1.0 ng/mL and level of expression of miR-137 corresponding to at least 5-fold increase compared to the control.

In the most preferred embodiment of this invention the subject is classified as high-risk early psychotic disorder subject based on expression level of COX6A2 below 1.2 ng/mL and level of expression of miR-137 corresponding to at least 2-fold increase corresponding to the healthy subject control. In other words, in the most preferred embodiment of this invention the subject is classified as high-risk early psychotic disorder subject based on expression level of COX6A2 below 1.2 ng/mL and level of expression of miR-137 higher than 4.8 a.u., as measured corresponding to the average expression level of miR-16, snRNA-U1 and snRNA-U6.

Based on the unexpected finding that expression levels of both biomarkers miR-137 and COX6A2 are correlated for early psychosis subjects but not for early controls, it was possible to propose the course of treatment of early psychosis subjects that would mechanistically reverse observed changes in biomarker levels. As disclosed herein observed changes in biomarker levels are indicative of oxidative stress in mitochondria, an agent useful for reversing the oxidative stress in mitochondria can plausibly be useful in treating the early psychosis disorder in subjects classified according to methods disclosed herein. Such course of treatment would not be obvious to a person skilled in the art. Thus, another aspect of the invention relates to a mitochondria-targeted antioxidant for use in the treatment of a subject classified as high risk psychotic disorder subject, preferably a high-risk schizophrenia subject, using the methods of this invention.

It is further noted that, as suggested by experiments in the animal models, expression levels of both biomarkers miR-137 and COX6A2 may be correlated for early psychosis subjects not only when determined in blood samples, but also when determined in the brain.

Antioxidant is broadly understood as a compound, substance or composition that can prevent oxidation processes, for example inside the cell, by reducing the concentration of available reactive oxygen species (ROS). Antioxidants comprise of structural features that can donate electrons. One example of such feature is quinone moiety, as shown here:

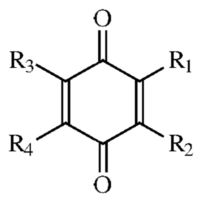

wherein $R_1$, $R_2$, $R_3$ and $R_4$ can be any substituents.

Mitochondria are subcellular compartments within the eukaryotic cells. Their abundance within the cell varies with the type of cell, stage of the cell cycles as well as proliferative stage of the cell. Their function lies within the generation of energy in the aerobic mode. Mitochondria-targeting molecules are the molecules that are preferably deposited within mitochondria, due to their structural features. Mitochondria have been shown to feature a strong proton gradient across the membrane that separates them from the cytoplasm. It is therefore known to that skilled in the art that positively charged molecules, that means the molecules that bear cationic charge, preferentially deposit themselves in mitochondria.

One example of a molecule that bears positive cationic charge and acts as an antioxidant thanks to the presence of quinone moiety is the compound of formula:

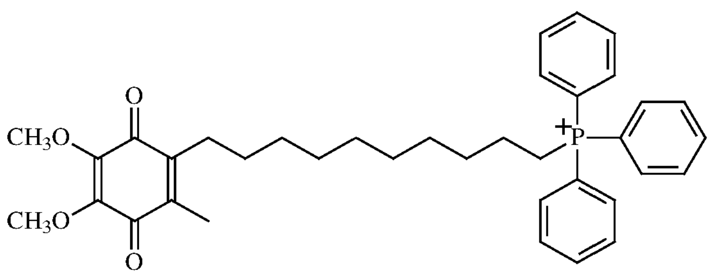

which is known to a person skilled in the art, and is commonly referred to as MitoQ. MitoQ is useful as an oxidant that is selectively deposited in mitochondria, as it can scavenge the reactive oxygen species in these subcellular compartments.

As preferably understood herein, mitochondria-targeted antioxidant can be provided as a pharmaceutically acceptable salt. "Pharmaceutically acceptable salts" are defined as derivatives of the disclosed compounds wherein the

parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445.

Preferably, within the scope of the present invention, MitoQ is present as a pharmaceutically acceptable salt, as defined herein. In a preferred embodiment, MitoQ is present as a methylsulfonate (also referred to as mesylate) salt.

It has been shown that transgenic mice carrying a permanent deficit of GSH synthesis (referred to as *Gclm*-KO) are a viable model of oxidative stress to schizophrenia. These mice exhibit widespread cortical deficiencies of parvalbumin interneurons and alterations of beta/gamma oscillations. Furthermore, when these mice that are exposed to GBR12909, a specific dopamine reuptake inhibitor that is useful to mimic the increased dopamine signaling observed in schizophrenia, the elevated levels of the oxidative DNA damage marker 8-oxo-7,8-dihydro-2'-deoxyguanosine is observed in the extranuclear compartment. It is indicative of mitochondrial DNA oxidation. These animals also exhibit increased levels of globular and dark mitochondria in neuropiles of parvalbumin interneurons, which is further consistent with inefficient clearance of damaged mitochondria through mitophagy.

It has been unexpectedly found that treatment with MitoQ, a selective mitochondria targeted-antioxidant, could rescue these deficits in young KO mice. Treatment with MitoQ (P21-P40) in *Gclm-KO* mice treated with GBR12909 normalizes NIX, FUNDC1 and LC3B expression levels as well as miR-137 expression intensity when compared to values observed for wild-type mice (Figure 7). Additionally, abnormalities in cell body numbers and staining intensity of parvalbumin interneuronal processes in the Anterior Cingulate Cortex of *Gclm-KO* mice treated with GBR12909 were also restored to wild type levels following treatment with MitoQ. Thus, MitoQ has been unexpectedly found to be useful in reversing the miR-137 overexpression, mitophagy and parvalbumin interneuron impairment induced by oxidative stress. It further shows that the upregulation of miR-137 and subsequent mitophagy defect constitutes the molecular mechanism underlying the oxidative stress induced parvalbumin interneuron impairment.

Further preferred aspect of this invention refers to a an embodiment, wherein the mitochondria-targeted antioxidant is selected from the group consisting of mitoquinone (MitoQ), 3-demethoxymitoquinone (DMMQ), 10-(6-plastoquinonyl) decyltriphenylphosphonium (SkQ1), SkQ3, coenzyme Q10 (CoQ10), methylene blue (MB). As understood to the skilled person, in certain embodiments the mitochondria-targeted antioxidant may preferably be present as a pharmaceutically acceptable salt thereof.

It is plausible that any compound or composition that is useful as antioxidant that can accumulate within the mitochondria can be useful in reversing the effects of oxidative damage of mitochondria and in treating the subjects that are classified as high-risk psychotic disorder subjects.

Further aspect of this invention relates to a biomarker kit comprising reagents for determining miR-137 and COX6A2 protein expression levels, using the methods disclosed herein. The reagents of the kit may be packages separately, selected reagents may also be packaged together or premixed. Quantification of the biomarkers used herein may be accomplished by any of a variety of established *in vitro* techniques that are useful for this purpose, known to that skilled in the art. These techniques may include, but are not limited to,; an mRNA expression level assay; *in situ* hybridization assay; a proteomics-based assay; a PCR-based assay; a real time PCR-based assay, next generation sequencing; an electrochemistry-based assay; a lateral-flow assay; a nanobead-based assay; a microfluidics-based assay; and an oligonucleotide-templated reaction.

In one embodiment, the kit provides the required reagents to perform RT PCR assay to perform quantification of miR-137 biomarker by using reverse transcription followed by quantitative polymerase chain reaction. To this end, the kit would include the reagents such as the reverse transcriptase, DNA polymerase, relevant buffers and deoxyribonucleotides, RT PCR detection dye and primers - short oligodeoxyribonucleotides suitable for starting the PCR reaction with miR-137 reverse transcription product as a template, as well as instructions on how to combine the reagents and perform the measurements. One possible way of performing the measurements is described in Examples.

In one embodiment, the kit provides the required reagents to perform the ELISA assay to quantify the cellular amount of COX6A2. In preferred embodiment, the kit contains required plates to serve as reaction vessel, antibodies for

capture and detection. The capture antibody is already precoated onto the plate, and the detection antibody is configured in such a way that capture and detection antibody can bind simultaneously to the same molecule of COX6A2. Detection antibody is further configured so that it either comprises a detection system, e.g. horseradish peroxidase, or can be coupled to detection system by using secondary antibody that comprises detection system, e.g. horseradish peroxidase. Kit additionally contains all the required buffers as well as instructions on how to mix reagents and perform the measurements.

Thus, the present invention relates to a kit as defined herein, wherein the COX6A2 expression level is determined as protein expression level.

**Brief description of the figures:**

Fig. 1 represents the correlation between the expression level of miR137 and expression level of COX6A2 in differently classified groups of patients.

Fig. 2 shows performance of the use of miR-137 biomarker alone and in combination with COX6A2, NIX, LC3B, FUNDC1, MMP9 and sRAGE, presented as the Receiver Operating Characteristic (ROC) curves.

Fig. 3 shows a Table summarizing the data on early psychosis patients that have been included in the study to validate the biomarkers of this invention.

Fig. 4 illustrates the measurement of miR-137 expression level in subjects, and provides comparison data for early psychotic patient group and control group.

Fig. 5 shows a patient selection method using the two biomarkers miR-137 and COX6A2 in the population of patients classified based on the polymorphism of miR-137 gene.

Fig. 6 shows a patient selection method using the two biomarkers miR-137 and COX6A2 in the population of patients classified based on the clinical factors. Combined use of miR-137 and COX6A2 levels allows classification of subjects that are eligible for MitoQ treatment without miR-137 SNP rs1625579 genotype consideration. (A) Identification of high-risk psychosis subjects based on miR-137 expression levels. (B) Identification of high-risk psychosis subjects based on COX6A2 protein levels. (C) Identification of high-risk psychosis patients based on combined detection of miR-137 and COX6A2 levels.

Fig. 7 shows changes in expression levels of miR-137 as well as mitophagy markers NIX, FUNDC1 and LC3, upon inducing oxidative stress through knockout of *Gclm* gene (which mediates biosynthesis of glutathione) and treatment with compound GBR12909, and rescue of observed effect through treatment with MitoQ, a mitochondria-targeted antioxidant (as discussed in Examples 9 and 10).

Fig. 8 presents the comparison of clinical psychosis parameters between the group of subjects classified as high-risk psychotic disorder subject and healthy subjects, based on the methods disclosed herein. High risk psychotic disorder subjects exhibit more significant positive symptoms, lower functional outcome (GAF) and more severe cognitive impairment, which is measured by assessing processing speed, attention, vigilance, working memory and verbal learning).

The examples below present different embodiments of this invention and their application to practice.

**Example 1** relates to the embodiments of this invention, wherein methods disclosed herein have been validated using a group of patients described below:

All the studies utilizing patient data or samples have been collected using the group of patient diagnosed as early psychotic disorder subjects based on clinical factors, whose population has been summarized in Fig. 3. The study population included early psychosis patients (EPP; *n* = 138) and healthy controls (*n* = 134), matched for gender and age. Patients were recruited from the Treatment and Early Intervention in Psychosis Program (TIPP, Lausanne University Hospital, Switzerland [1], which is a specialized 3-year program for the treatment of early psychosis patients. Inclusion criteria were: (i) individuals aged 18 to 35 years old; (ii) residence in Lausanne and surroundings areas; (iii) meeting threshold criteria for psychosis, as defined by the 'Psychosis threshold' subscale of the Comprehensive Assessment of At Risk Mental State (CAARMS) [2]; (iv) no more than 6 months of treatment with antipsychotic medication for psychosis; (v) no psychosis related to intoxication or organic brain disease; (vi) intelligence quotient $\geq$ 70; and (vii) ability to discern and to provide informed consent.

The psychosis threshold and the diagnosis assessment resulted from an expert consensus including a senior psychiatrist, a psychologist and the case manager who closely followed-up the patient during the 3-year program. The duration of the illness considered the time elapsed from the psychosis threshold to the participation in the study and the diagnosis was based on DSM-IV criteria (American Psychiatric Association, 1994). Most of the patients (*n* = 123) had antipsychotic medication (374.2 $\pm$ 216.5 mg chlorpromazine equivalent dose (CPZ); It is noteworthy that patients who accepted to participate in the present study were representative of the entire clinical TIPP cohort. Healthy controls were

recruited within same catchment area. They were assessed by the Diagnostic Interview for Genetic Studies (DIGS) in order to attest for the absence of any major psychiatric or substance use disorder. In addition, healthy controls who reported having a first-degree relative with psychotic disorder were excluded. Neurological disorders and severe head trauma were also exclusion criteria for all subjects.

**Example 2** of this invention refers to an embodiment, wherein samples for determination of expression levels of biomarkers are isolated from exosomes, obtained from peripheral blood sample. For the isolation of exosomes from human plasma, 200-400 μl of plasma was separated by ultracentrifugation (Sorvall ultra Pro 80).

**Example 3** relates to an embodiment, wherein expression level of circulating miR-137 is performed by using RT-PCR protocol, according to the following procedure:

miRNAs were extracted from exosome using miRNeasy Kit (Qiagen, Hilden, Germany). The protocol used was provided with the kit and described in "miRNeasy Handbook". The quality of extracted miRNAs and their concentration were determined with a NanoDrop (ND-1000 spectrophotometer, Thermo Fisher Scientific, USA) by measuring the absorbance at 260 nm (A260) and 280 nm (A280). The A260/A280 ratio had to be ~2.0 for pure miRNA (as for pure RNA).

For first-strand cDNA synthesis by reverse transcription, total purified miRNA samples were diluted to 5 ng/μl. miRNA was reverse transcribed using the miRCURY LNA Universal cDNA Synthesis kit (Exiqon, Vedbaek, Denmark) according to the instructions enclosed. A mixture containing 4μl of total miRNA, 2μl of the Enzyme mix, and 4μl of the 5x Reaction buffer concentrate was made up to 20μl final volume with nuclease-free water. The cocktail was gently vortexed to thoroughly mix all reagents. Final solution was spun down and incubated for reverse transcription at 42°C for 60 minutes, followed by 5 minutes at 95°C. The obtained cDNA templates were immediately cooled on ice and stored at 4° C.

For qPCR amplification the cDNA templates and Exiqon Master mix (Exiqon, Vedbaek, Denmark) were used, following the product user instructions. This procedure suggests the use of three replicas for each plasma sample and for each miRNA. For the amplification, final reaction volumes of 10μl were prepared in the following proportions: 5μl of master mix, 1μl of LNA primer set (0.5μl of each forward and reverse primers) and 4μl of 1.25% solution of the cDNA template in nuclease free water. qPCR amplification was performed with a qPCR Detection System with the thermal cycling parameters: 10 minutes at 95°C, 50 cycles (10 sec. each) at 95°C, 60°C for one minute. qPCR values (quantification cycle "Cq") for studied miRNAs were normalized to three reference miRNAs (miR-Ref) miR-16, snRNA-U1 and snRNA-U6

**Example 4** relates to an embodiment, wherein the plasmatic level of COX6A2 is determined by using the Western Blot technique, according to the following protocol:

Total proteins were loaded on 12.5% acrylamide gels for SDS-PAGE and transferred to nitrocellulose membranes. Membranes were blocked in Tris Buffer Saline with 2% non-fat dried milk and hybridized overnight at 4°C with a primary antibody diluted in blocking buffer (anti-COX6A2, rabbit, dilution 1:500). Proteins on immunoblots were revealed using appropriate secondary antibody conjugated to Horseradish peroxidase (HRP) corresponding to the primary antibody (anti-rabbit). Images were acquired and quantified using Fusion Imaging System (Fusion Solo-S). The comparison between the samples that originate from early psychotic disorder subjects and the control group is shown in Figure 4.

**Example 5** relates to an embodiment of present invention, wherein the plasmatic expression level of COX6A2 is determined by using the ELISA (Enzyme-Linked Immunosorbent Assay) technique, according to the following protocol: ELISA Kit for COX6A2 detection was used according the provided instructions (Human Cytochrome c oxidase subunit 6A2, mitochondrial (COX6A2) ELISA Kit). Briefly, the assay sample and buffer are incubated in a pre-coated plate, together with COX6A2-HRP conjugate for one hour. After the incubation and several washes the HRP substrate [TMB (3,3',5,5'-tetramethylbenzidine)] is added. The intensity of color is measured using a spectrophotometer Tecan (Vitaris AG). A standard curve is plotted relating the intensity of the color to the concentration of standards. This standard curve is used to define COX6A2 level in each sample.

**Example 6** relates to the embodiment of this invention, wherein selection of subjects is performed based on the expression levels of miR-137 and COX6A2, and the successful classification is checked against available genetic data that predisposes subjects to be high-risk schizophrenia subjects (Figure 5). First, in the step (A) the subjects were selected based on expression level of miR-137, higher than the threshold of 4.8 a.u.. In the second step, the expression level of COX6A2 was examined, and subjects with expression level lower than 1.2 ng/mL have been excluded from the subject group (B). It should be noted that the correlation between the genetic biomarkers and molecular biomarkers further makes plausible the application of both biomarkers in the clinic.

**Example 7** relates to an embodiment of this invention as in Example 6, with the difference that biomarker classification is checked against available clinical data, that is, classification of patients as high-risk psychotic disorder subjects according to the patient selection of Example 1. Example 7 further illustrates how combined use of both biomarkers miR-137 and can be used to exclude healthy subjects. Figure 6 presents the details of performed classification. The left panel shows classification of two groups of subjects - one group containing early psychosis subjects (left) and the second group containing the healthy subjects (control group) - according to the expression level of miR-137. Most of the early psychosis subject group (68%) is included based on the expression level of miR-137 higher than the threshold (4.8 a.u. as normalized to miR-16, snRNA-U1 and snRNA-U6 expression levels). At the same time, only 17% of healthy subjects can be excluded based on the miR-137 expression level threshold of 4.8 a.u.. The right panel of Figure 6 presents

further classification of patients based on the expression level of COX6A2. While most of the subjects in early psychotic subject group that were selected based on miR-137 expression level also fulfilled the COX6A2 expression level requirements (set us lower than 1.2 ng/mL), there was significantly less correlation between the two parameters in the control group. Hence, 25% of control subjects that fulfill the criterion of miR-137 expression higher than threshold could be excluded based on the expression level of COX6A2.

**Example 8** relates to the study of Examples 6 and 7, wherein the correlation between the expression level of miR-137 and the expression level of COX6A2 has been studied for different groups of subject. Figure 1 clearly shows the correlation between both biomarker expression level for the group of high risk early psychotic disorder subjects, in both classification cases, disregarding genetic factors (p= 0.0062 and R=0.532) and taking them into account (p = 0.0014, R=0.547). At the same time, no significant correlation was observed for the control subject group, in both classification cases, disregarding genetic factors (p=0.51, R=0.090) and taking them into account (p=0.72, R=0.049).

**Example 9** relates to an animal model of oxidative stress. Oxidative stress elicits mitophagy in the anterior cingulated cortex (ACC) of *Gclm-KO* mice (transgenic mice carrying permanent defect in glutathione GSH synthesis). Pharmacological challenge of *Gclm-KO* mice with GBR12909 (*Gclm*-KO+GBR) is known to induce elevated levels of the oxidative DNA damage marker 8-oxo-7,8-dihydro-2'-deoxyguanosine (8-oxo-dG) in the ACC of these animals. Because extranuclear localization of 8-oxo-dG labeling was observed, it could be surmised that mitochondrial DNA (mtDNA) was affected. Developmental oxidative stress resulted in abnormal mitochondrial morphology in the ACC of *Gclm*-KO+GBR (P20) as opposed to wild type (WT) animals. Specifically, qualitative assessment of electron micrographs revealed increased numbers of mitochondria with darker and a globular shape suggestive of impaired mitophagy. Principal markers of mitophagy including NIX, Fundc1 and LC3B were found to be significantly decreased in the *Gclm*-KO+GBR compared to WT mice (see Figure 7 for details). In comparison with WT mice (*Gclm*-WT+PBS, wherein PBS refers to phosphate-buffered saline, a common buffer used in these experiments as vehicle), levels of the mitophagy receptors NIX and FUNDC1 and of the autophagosome LC3B were reduced in the ACC of *Gclm-KO* mice (*Gclm*-KO+PBS, at P40). Additional oxidative insult with GBR12909 (administered between P10-P20) further decreased NIX, FUNDC1 and LC3B staining intensity in *Gclm*-KO+GBR mice). Taken together, these observations are consistent with an oxidative stress-induced mitophagy deficit in prefrontal parvalbumin interneurons.

It has been shown that miR-137 plays a functional role in modulating synaptic function and in regulating the expression of the NIX and FUNDC1 mitophagy receptors. Combined *in situ* hybridization and immunohistochemistry was therefore applied to quantify the expression levels of miR-137 within the ACC, showing that it was significantly increased in *Gclm-KO* mice (Figure 7). Additional oxidative challenge with GBR (administered between P10-P20) in *Gclm-KO* mice induced a marked increase in the co-localization of miR-137 staining in PVIs within the ACC at P40, suggesting that the elevated expression of miR-137 observed in the ACC was localized to parvalbumin interneurons. Consistent with previous results, parvalbumin immunoreactive cell count and puncta intensity were decreased in *Gclm-KO* and were further depleted in *Gclm*-KO+GBR animals. These findings suggest that in animals with genetically induced redox dysregulation, which are challenged with an additional environmental insult (i.e., *Gclm*-KO+GBR) there is upregulation of miR-137, which leads to decreased mitophagy and a subsequent accumulation of damaged mitochondria that further exacerbates oxidative stress and parvalbumin impairment.

**Example 10** relates to mouse model experiment which demonstrates that treatment with MitoQ rescues oxidative stress induced elevated level of miR-137 expression, mitophagy, and parvalbumin interneurons alterations. In view of the detrimental effects of oxidative stress on integrity of parvalbumin interneurons, miR-137 expression level as well as mitophagy during preweaning and pubertal stages of development, it was evaluated whether MitoQ treatment, a selective mitochondria targeted-antioxidant, could rescue these deficits in young KO mice. Treatment with MitoQ (P21-P40) in *Gclm*-KO+GBR mice normalized NIX, FUNDC1 and LC3B expression levels as well as miR-137 expression intensity, when compared to WT values (Figure 7). Additionally, abnormalities in cell body numbers and staining intensity of parvalbumin interneuron processes in the ACC of *Gclm*-KO+GBR animals were similarly restored to WT levels following treatment with MitoQ. Thus, MitoQ can reverse the oxidative stress induced miR-137 overexpression, mitophagy and parvalbumin interneuron impairment, highlighting that the upregulation of miR-137 and subsequent mitophagy defect constitutes the molecular mechanism underlying the oxidative stress-induced parvalbumin interneuron impairment.

**Example 11** relates to the study of correlation between the high risk psychotic disorder classification based on the measurement of the expression level of miR-137 and COX6A2, and clinical indicators of psychosis. As shown in Figure 8, high risk psychotic disorder subjects exhibit more significant positive symptoms, lower functional outcome (GAF) and more severe cognitive impairment, which is measured by assessing processing speed, attention, vigilance, working memory and verbal learning).

## Claims

**1.** A method for classifying a subject as high-risk psychotic disorder subject, the method comprising the steps of:

(a) providing a biological sample obtained from a subject;
(b) determining the expression level of miRNA-137, and the expression level of COX6A2; and
(c) classifying the subject as high-risk psychotic disorder subject based on expression levels determined in step (b),

wherein the psychotic disorder is schizophrenia.

2. The method of claim 1, wherein the psychotic disorder is early stage schizophrenia

3. The method of claim 1 or 2, wherein the psychotic disorder is early stage schizophrenia with cognitive impairment.

4. The method of claim 3, wherein the classification comprises differential classification between high-risk schizophrenia subjects and low-risk schizophrenia subjects.

5. The method of any one of claims 1 to 4, wherein the biological sample comprises peripheral blood, plasma, serum, cerebrospinal fluid, blood-derived exosomes, plasma-derived exosomes, neural exosomes, cortical tissue, post-mortem brain tissue, fibroblast cell culture, induced pluripotent stem cell culture, derived neuronal precursor cell culture.

6. The method of any one of the claims 1 to 5, wherein the relative expression level of miRNA-137 normalised to reference genes is higher than a threshold value.

7. The method of claim 6, wherein the threshold value is 4.8 a.u, as normalized with respect to the average expression level of miR-16, snRNA-U1 and snRNA-U6.

8. The method of claim 6, wherein the threshold value corresponds to at least twofold increase of miR-137 expression level as compared with the healthy subjects.

9. The method of any one of claims 1 to 8, wherein the expression level of COX6A2 is lower than a threshold value.

10. The method of claim 9, wherein the threshold value is 1.2 ng/ml.

11. The method of any one of claims 1 to 10, wherein expression level of miR-137 and expression level of COX6A2 are determined by an in vitro assay, wherein the in vitro assay is selected from the group consisting of an immunoassay; an ELISA-based assay, an aptamer-based assay; an mRNA expression level assay; in situ hybridization assay; a proteomics-based assay; a PCR-based assay; a real time PCR-based assay, next generation sequencing; an electrochemistry-based assay; a lateral-flow assay; a nanobead-based assay; a microfluidics-based assay; and an oligonucleotide-templated reaction.

12. A mitochondria-targeted antioxidant for use in the treatment of a subject classified as high risk schizophrenia subject, using the method of any one of claims 1-11.

13. The mitochondria-targeted antioxidant for use of claim 12, wherein the mitochondria-targeted antioxidant is selected from the group consisting of mitoquinone (MitoQ), 3-demethoxymitoquinone (DMMQ), 10-(6-plastoquinonyl) decyltriphenylphosphonium (SkQ1), SkQ3, coenzyme Q10 (CoQ10), methylene blue (MB).

14. A biomarker kit comprising the required reagents for determining miR-137 and COX6A2 expression levels as defined in any one of claims 1 to 11, wherein the COX6A2 expression level is determined as protein expression level.

## Patentansprüche

1. Verfahren zur Klassifizierung eines Subjekts als Subjekt mit hohem Risiko einer psychotischen Störung, wobei das Verfahren die Schritte umfasst:

(a) Bereitstellen einer biologischen Probe, die von einem Subjekt erhalten wurde;
(b) Bestimmen des Expressionsniveaus von miRNA-137 und des Expressionsniveaus von COX6A2; und
(c) Klassifizieren des Subjekts als Subjekt mit hohem Risiko einer psychotischen Störung auf Grundlage der in

Schritt (b) bestimmten Expressionsniveaus, wobei die psychotische Störung Schizophrenie ist.

2. Verfahren nach Anspruch 1, wobei die psychotische Störung ein frühes Stadium der Schizophrenie ist.

3. Verfahren nach Anspruch 1 oder 2, wobei die psychotische Störung ein frühes Stadium der Schizophrenie mit kognitiver Beeinträchtigung ist.

4. Verfahren nach Anspruch 3, wobei die Klassifizierung die differenzielle Klassifizierung zwischen Subjekten mit hohem Risiko von Schizophrenie und Subjekten mit geringem Risiko von Schizophrenie umfasst.

5. Verfahren nach einem der Ansprüche 1 bis 4, wobei die biologische Probe peripheres Blut, Plasma, Serum, zerebrospinale Flüssigkeit, von Blut abgeleitete Exosome, von Plasma abgeleitete Exosome, neuronale Exosome, kortikales Gewebe, Post-Mortem-Gehirngewebe, Fibroblasten-Zellkultur, induzierte pluripotente Stammzell-Kultur, abgeleitete neuronale Vorläufer-Zellkultur umfasst.

6. Verfahren nach einem der Ansprüche 1 bis 5, wobei das relative Expressionsniveau von miRNA-137 normalisiert in Bezug auf Referenzgene höher ist als ein Schwellenwert.

7. Verfahren nach Anspruch 6, wobei der Schwellenwert normalisiert in Bezug auf das durchschnittliche Expressionsniveau von miR-16, snRNA-U1 und snRNA-U6 4,8 a.u. beträgt.

8. Verfahren nach Anspruch 6, wobei der Schwellenwert zumindest einer zweifachen Erhöhung des Expressionsniveaus von miR-137 verglichen mit gesunden Subjekten entspricht.

9. Verfahren nach einem der Ansprüche 1 bis 8, wobei das Expressionsniveau von COX6A2 niedriger als ein Schwellenwert ist.

10. Verfahren nach Anspruch 9, wobei der Schwellenwert 1,2 ng/ml beträgt.

11. Verfahren nach einem der Ansprüche 1 bis 10, wobei das Expressionsniveau von miR-137 und das Expressionsniveau von COX6A2 durch einen in-vitro-Assay bestimmt werden, wobei der in vitro-Assay ausgewählt ist aus der Gruppe bestehend aus einem Immunassay, einem ELISA-basierten Assay, einem Aptamer-basierten Assay, einem mRNA-Expressionsniveau-Assay, einem in-situ-Hybridisierungs-Assay, einem Proteomik-basierten Assay, einem PCR-basierten Assay, einem Echtzeit-PCR-basierten Assay, der Next-Generation-Sequenzierung, einem Elektrochemie-basierten Assay, einem Lateral-Flow-Assay, einem Nanokügelchen-basierten Assay, einem Mikrofluidik-basierten Assay und einer Oligonukleotid-Template-Reaktion.

12. Auf Mitochondrien abzielendes Antioxidans zur Verwendung bei der Behandlung eines Subjekts, das als Subjekt mit hohem Risiko für Schizophrenie klassifiziert ist, unter Verwendung des Verfahrens nach einem der Ansprüche 1-11.

13. Auf Mitochondrien abzielendes Antioxidans zur Verwendung nach Anspruch 12, wobei das auf Mitochondrien abzielende Antioxidans ausgewählt ist aus der Gruppe bestehend aus Mitoquinon (MitoQ), 3-Demethoxymitoquinon (DMMQ), 10-(6-Plastoquinonyl)-decyltriphenylphosphonium (SkQ1), SkQ3, Coenzym Q10 (CoQ10), Methylenblau (MB).

14. Biomarker-Kit, umfassend die erforderlichen Reagenzien zur Bestimmung von Expressionsniveaus von miR-137 und COX6A2, wie in einem der Ansprüche 1 bis 11 definiert, wobei das COX6A2-Expressionsniveau als Proteinexpressionsniveau bestimmt wird.

## Revendications

1. Procédé pour classer un sujet comme sujet à haut risque de trouble psychotique, le procédé comprenant les étapes :

(a) de fourniture d’un échantillon biologique obtenu à partir d’un sujet ;
(b) de détermination du niveau d’expression du miARN-137 et du niveau d’expression de COX6A2 ; et
(c) de classification du sujet comme sujet à haut risque de trouble psychotique sur la base des niveaux d’expression déterminés dans l’étape (b),

dans lequel le trouble psychotique est la schizophrénie.

2. Procédé selon la revendication 1, dans lequel le trouble psychotique est une schizophrénie à un stade précoce.

3. Procédé selon la revendication 1 ou 2, dans lequel le trouble psychotique est une schizophrénie à un stade précoce avec déficience cognitive.

4. Procédé selon la revendication 3, dans lequel la classification comprend une classification différentielle entre les sujets à haut risque de schizophrénie et les sujets à faible risque de schizophrénie.

5. Procédé selon l'une quelconque des revendications 1 à 4, dans lequel l'échantillon biologique comprend du sang périphérique, du plasma, du sérum, du liquide céphalorachidien, des exosomes dérivés du sang, des exosomes dérivés du plasma, des exosomes neuronaux, du tissu cortical, du tissu cérébral post-mortem, une culture de cellules de fibroblastes, une culture de cellules souches pluripotentes induites, une culture de cellules précurseurs neuronales dérivées.

6. Procédé selon l'une quelconque des revendications 1 à 5, dans lequel le niveau d'expression relatif du miARN-137 normalisé par rapport aux gènes de référence est supérieur à une valeur seuil.

7. Procédé selon la revendication 6, dans lequel la valeur seuil est de 4,8 a.u, telle que normalisée par rapport au niveau d'expression moyen du miR-16, de l'ARNsn-U1 et de l'ARNsn-U6.

8. Procédé selon la revendication 6, dans lequel la valeur seuil correspond à une augmentation d'au moins deux fois du niveau d'expression de miR-137 par rapport aux sujets sains.

9. Procédé selon l'une quelconque des revendications 1 à 8, dans lequel le niveau d'expression de COX6A2 est inférieur à une valeur seuil.

10. Procédé selon la revendication 9, dans lequel la valeur seuil est de 1,2 ng/ml.

11. Procédé selon l'une quelconque des revendications 1 à 10, dans lequel le niveau d'expression de miR-137 et le niveau d'expression de COX6A2 sont déterminés par un dosage in vitro, où le dosage in vitro est choisi dans le groupe constitué par un dosage immunologique ; un dosage basé sur ELISA, un dosage à base d'aptamères ; un dosage de niveau d'expression d'ARNm ; un dosage d'hybridation in situ ; un dosage basé sur la protéomique ; un dosage basé sur la PCR ; un dosage basé sur la PCR en temps réel, le séquençage de nouvelle génération ; un dosage basé sur l'électrochimie ; un dosage à flux latéral ; un dosage basé sur des nanobilles ; un dosage basé sur la microfluidique ; et une réaction à matrice d'oligonucléotides.

12. Antioxydant ciblant les mitochondries pour une utilisation dans le traitement d'un sujet classé comme sujet à haut risque de schizophrénie, en utilisant le procédé de l'une quelconque des revendications 1 à 11.

13. Antioxydant ciblant les mitochondries pour une utilisation selon la revendication 12, dans lequel l'antioxydant ciblant les mitochondries est choisi dans le groupe constitué par la mitoquinone (MitoQ), la 3-déméthoxymitoquinone (DMMQ), le 10-(6-plastoquinonyl)décyltriphénylphosphonium (SkQ1), SkQ3, la coenzyme Q10 (CoQ10) et le bleu de méthylène (MB).

14. Kit de biomarqueurs comprenant les réactifs nécessaires pour déterminer les niveaux d'expression de miR-137 et COX6A2 tels que définis dans l'une quelconque des revendications 1 à 11, où le niveau d'expression de COX6A2 est déterminé en tant que niveau d'expression de protéine.

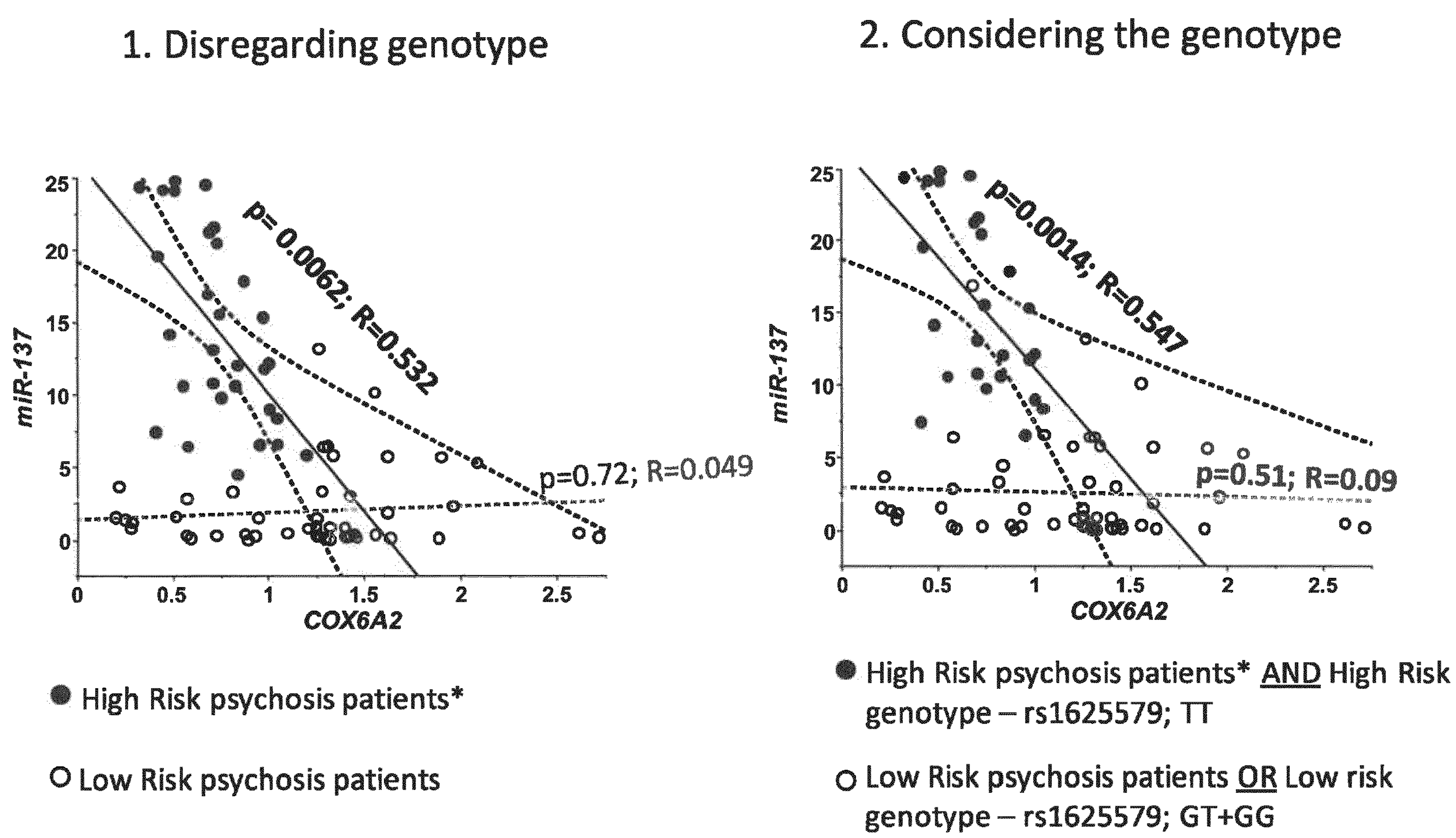


*Based on combined detection of miR-137 and COX6A2 levels in blood samples

Figure 1.

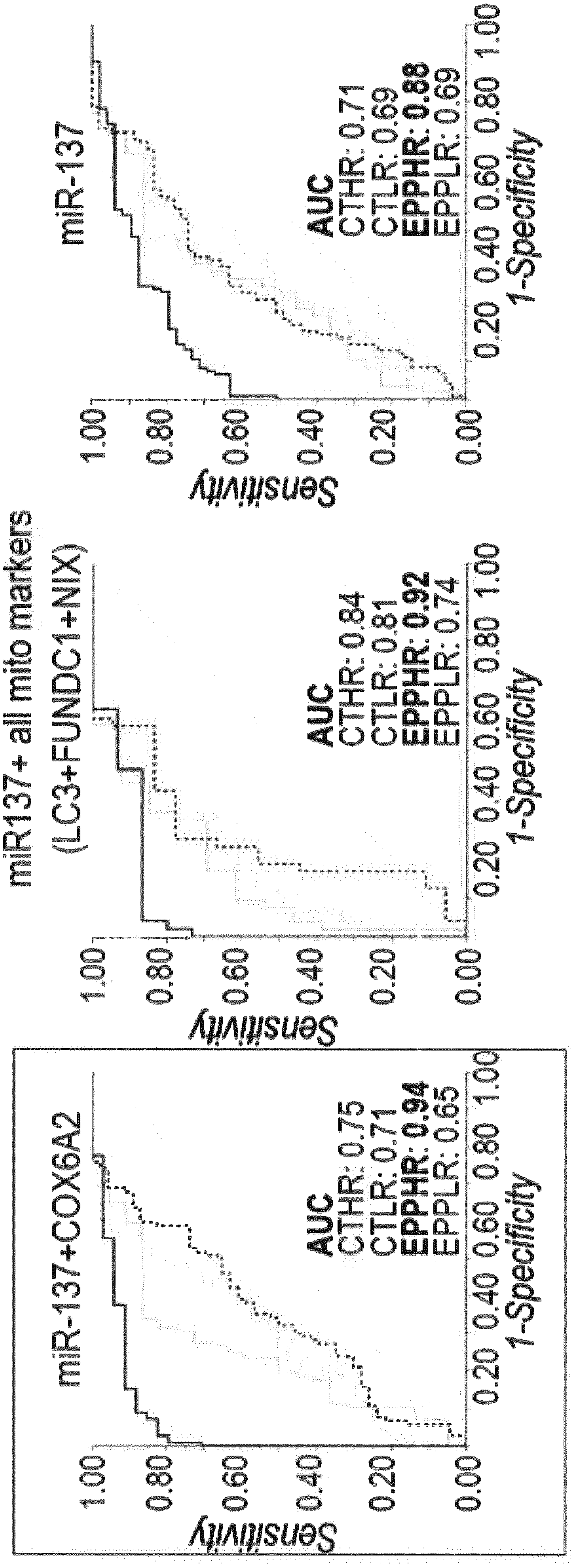
miR-137+COX6A2
Sensitivity
1.00
0.80
0.60
0.40
0.20
0.00
AUC
CTHR: 0.75
CTLR: 0.71
EPPHR: 0.94
EPPLR: 0.65
0.20 0.40 0.60 0.80 1.00
1-Specificity
miR137+ all mito markers
(LC3+FUNDC1+NIX)
Sensitivity
1.00
0.80
0.60
0.40
0.20
0.00
AUC
CTHR: 0.84
CTLR: 0.81
EPPHR: 0.92
EPPLR: 0.74
0.20 0.40 0.60 0.80 1.00
1-Specificity
miR-137
Sensitivity
1.00
0.80
0.60
0.40
0.20
0.00
AUC
CTHR: 0.71
CTLR: 0.69
EPPHR: 0.88
EPPLR: 0.69
0.20 0.40 0.60 0.80 1.00
1-Specificity

Figure 2.

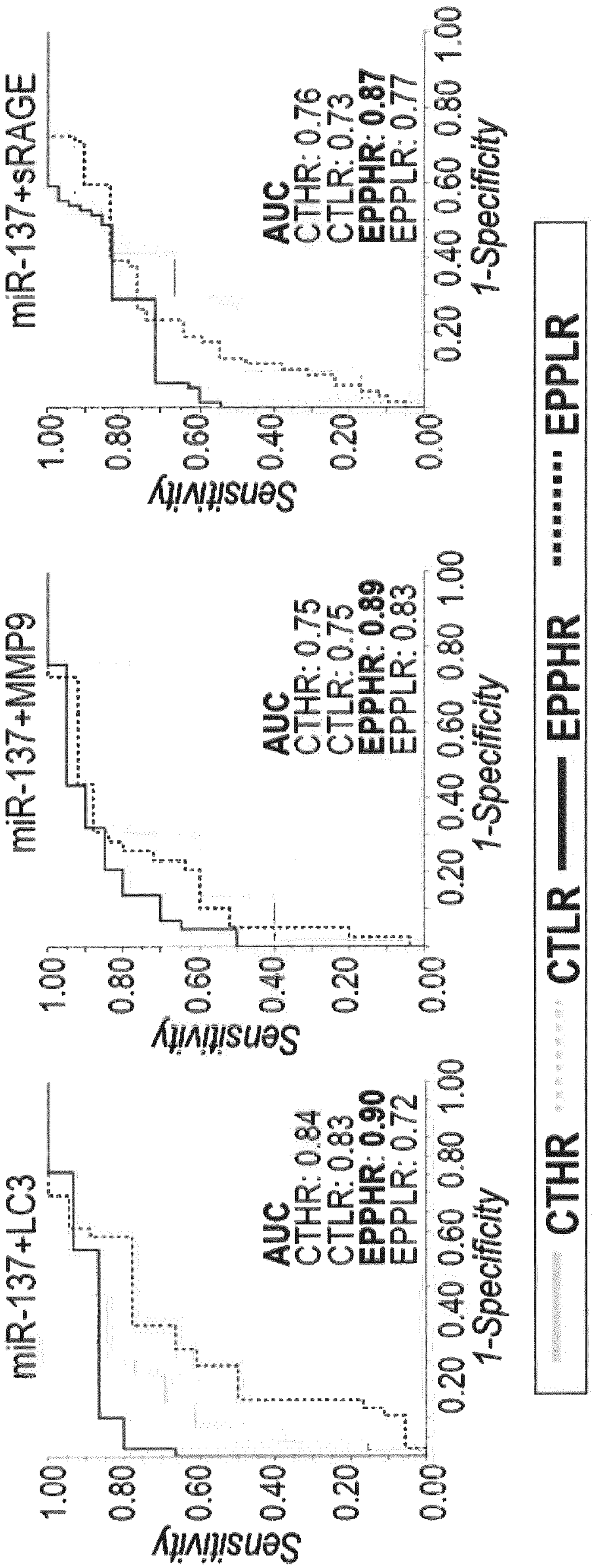


Figure 2 continued.

| | Main cohort | | | EEG cohort | | |
|---|---|---|---|---|---|---|
| | Controls N = 134 | EP Patients N = 138 | Test value | Controls N = 31 | EP Patients N = 30 | Test value |
| Age, mean ± SD, years | 25.5 ± 4.5 | 24.7 ± 4.6 | t (270) = 1.465 ; p = .144 | 25.5 ± 4.7 | 25.2 ± 3.9 | t (59) = 0.269 ; p = .789 |
| Gender, % of men (n) | 65.7 (134) | 73.9 (138) | $\chi^2$ (1) = 2.193 ; p = .139 | 74.2 (31) | 80.0 (30) | $\chi^2$ (1) = 0.291 ; p = .590 |
| Ethnicity, % of caucasien (n) | 83.1 (130) | 65.9 (138) | $\chi^2$ (1) = 10.281 ; p = .001 | 71.0 (31) | 60.0 (30) | $\chi^2$ (1) = 0.812 ; p = .367 |
| Handedness, % of right-handed (n) | 86.6 (119) | 89.9 (99) | $\chi^2$ (2) = 1.734 ; p = .420 | 87.1 (31) | 96.7 (30) | $\chi^2$ (1) = 1.856 ; p = .173 |
| Duration of illness, mean ± SD, days | - | 562.8 ± 472.1 | | - | 376.8 ± 359.6 | |
| Age at psychosis, mean ± SD, years | - | 23.1 ± 4.7 | | - | 24.1 ± 3.8 | |
| CPZ eq., mean ± SD, mg/day | - | 374.2 ± 216.5 | | - | 354.7 ± 202.7 | |
| PANSS Positive factor, mean ± SD | - | 7.2 ± 3.1 | | - | | |
| PANSS Negative factor, mean ± SD | - | 14.9 ± 5.8 | | - | | |
| PANSS Disorganized factor, mean ± SD | - | 5.9 ± 2.2 | | | | |
| PANSS Excited factor, mean ± SD | - | 6.1 ± 2.5 | | | | |
| PANSS Depressed factor, mean ± SD | - | 8.2 ± 3.3 | | - | | |
| GAF, mean ± SD | 83.6 ± 4.6 | 54.2 ± 13.0 | t (204) = 22.558 ; p < .001 | 84.8 ± 4.1 | 54.0 ± 11.4 | t (57) = 13.843 ; p < .001 |
| Diagnostic, % of cohort (n) | | | | | | |
| Bipolar disorder | | 10.1 (14) | | | 3.3 (1) | |
| Brief psychotic episode | | 14.5 (20) | | | 13.3 (4) | |
| MDD with psychotic features | | 2.2 (3) | | | 6.7 (2) | |
| Psychosis not otherwise specified | | 4.3 (6) | | | 3.3 (1) | |
| Schizoaffective disorder | | 8.7 (12) | | | 13.3 (4) | |
| Schizophrenia | | 60.1 (83) | | | 60.0 (18) | |
| EEG-ASSR-40, mean ± SD, Nb of epochs | | | | 132.3 ± 8.7 | 130.7 ± 9.9 | t (59) = 0.667 ; p = .507 |

Figure 3.

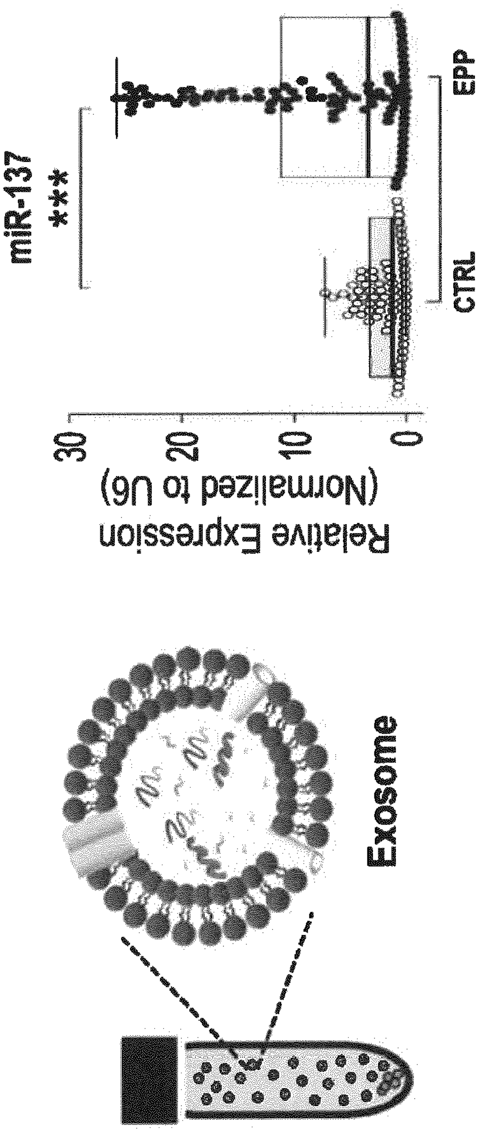
Exosome
miR-137
***
Relative Expression
(Normalized to U6)
30
20
10
0
CTRL
EPP

Figure 4.

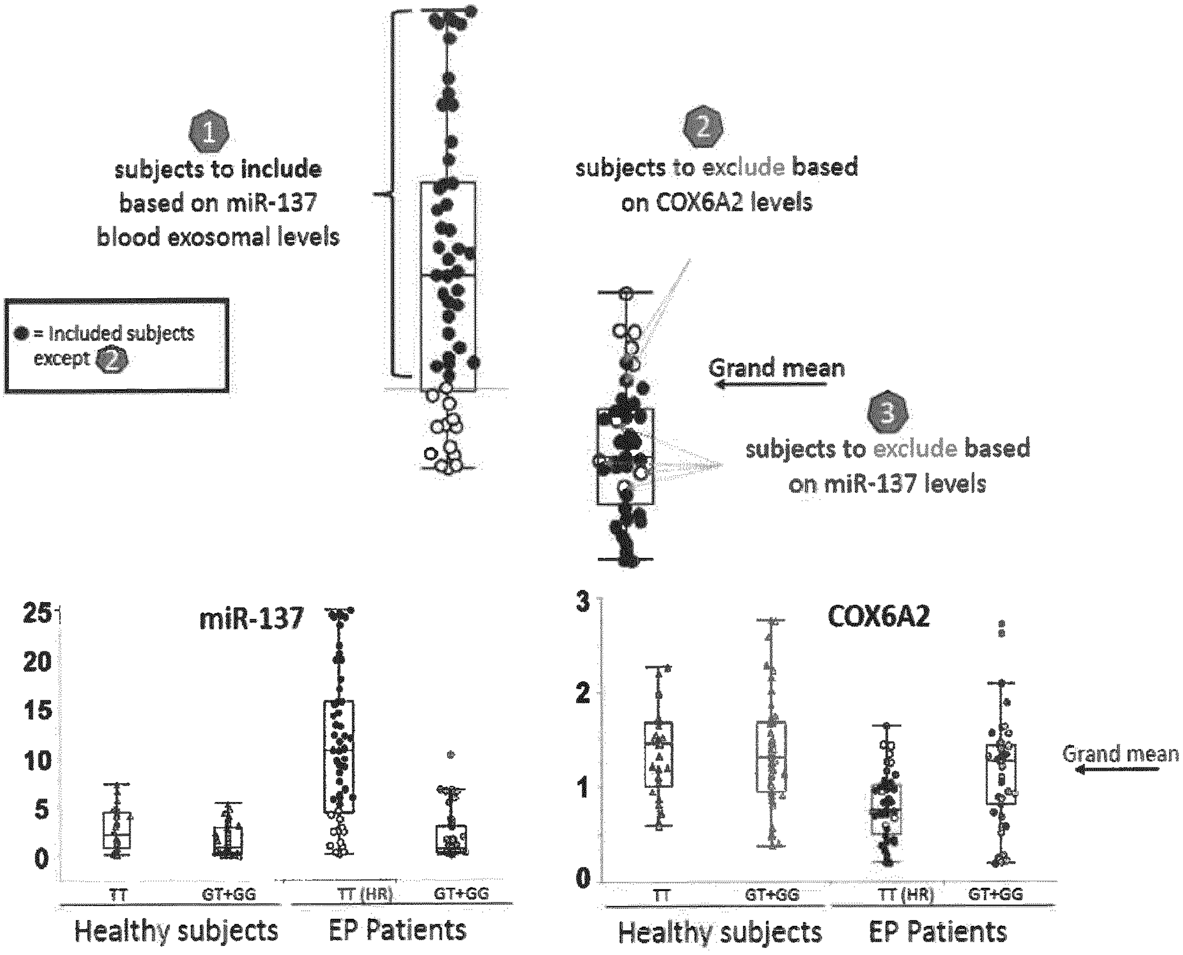
1
subjects to include based on miR-137 blood exosomal levels
● = Included subjects except 2
2
subjects to exclude based on COX6A2 levels
Grand mean
3
subjects to exclude based on miR-137 levels
25
20
15
10
5
0
miR-137
TT
GT+GG
TT (HR)
GT+GG
Healthy subjects
EP Patients
3
2
1
0
COX6A2
TT
GT+GG
TT (HR)
GT+GG
Grand mean
Healthy subjects
EP Patients

Figure 5.

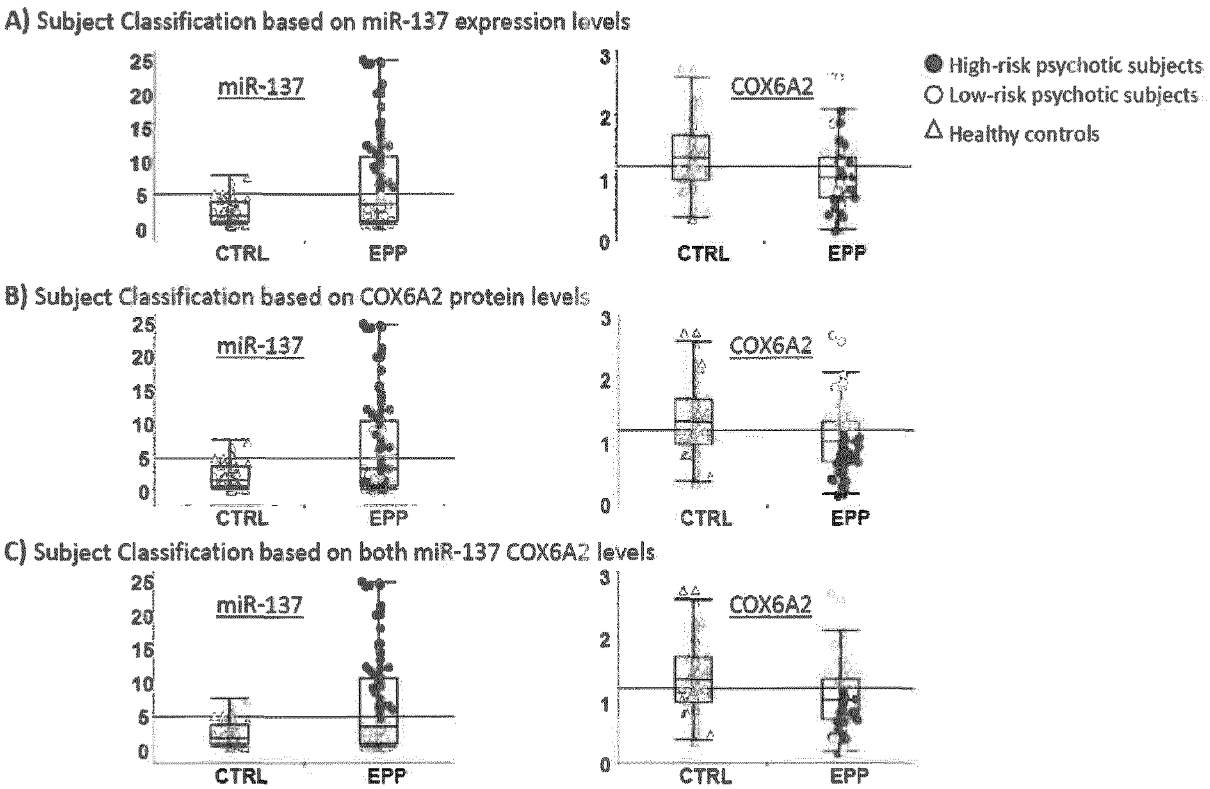
A) Subject Classification based on miR-137 expression levels
miR-137
COX6A2
CTRL
EPP
High-risk psychotic subjects
Low-risk psychotic subjects
Healthy controls
B) Subject Classification based on COX6A2 protein levels
C) Subject Classification based on both miR-137 COX6A2 levels

Figure 6.

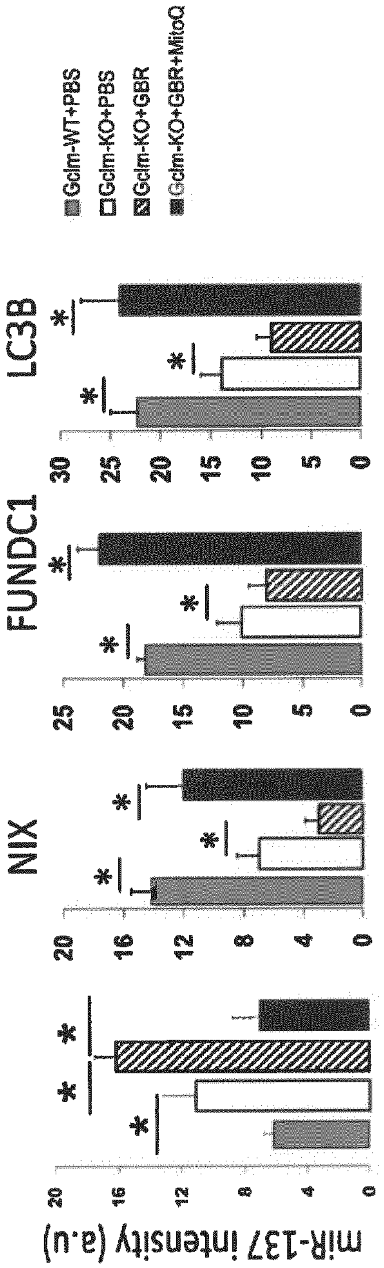

miR-137 intensity (a.u)
NIX
FUNDC1
LC3B
Gclm-WT+PBS
Gclm-KO+PBS
Gclm-KO+GBR
Gclm-KO+GBR+MitoQ


Figure 7.

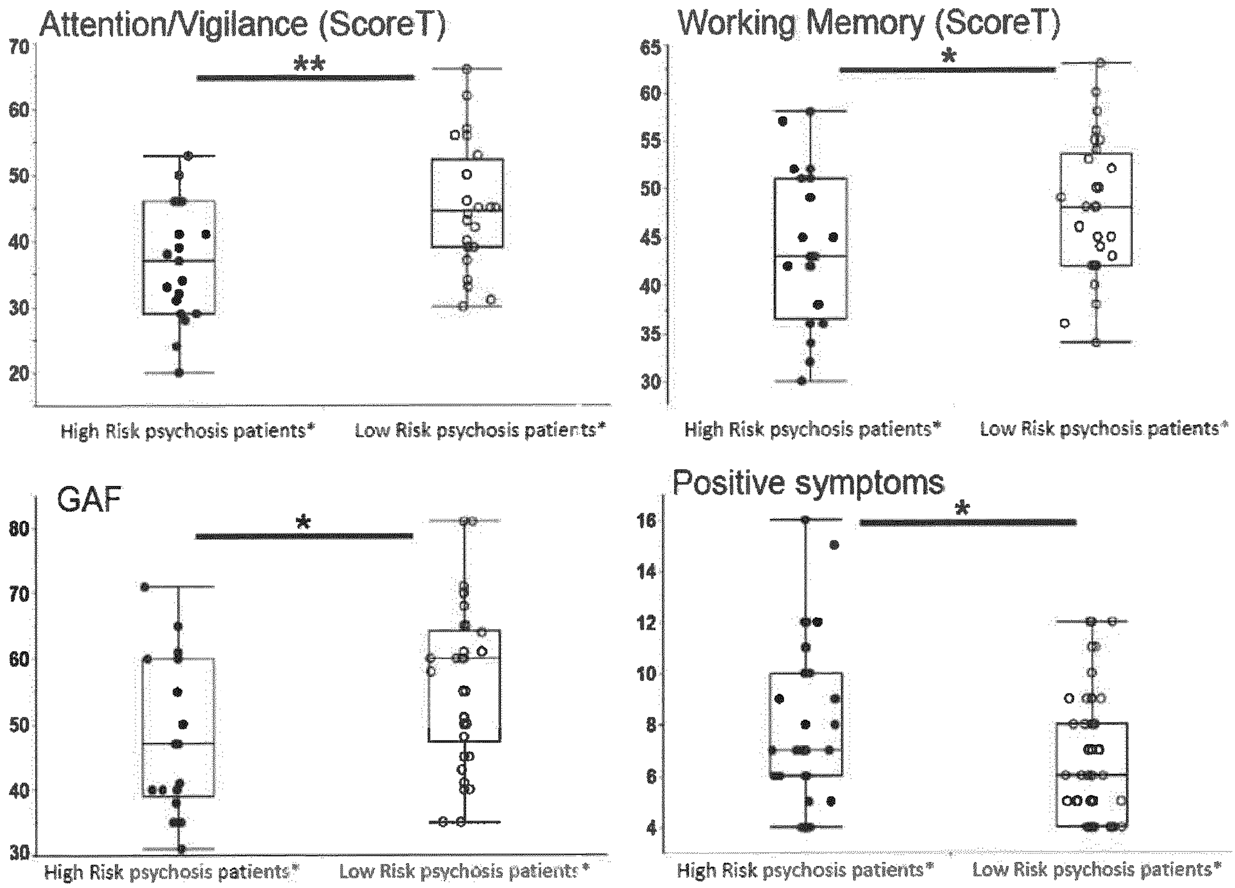
Attention/Vigilance (ScoreT)
**
70
60
50
40
30
20
High Risk psychosis patients*
Low Risk psychosis patients*
Working Memory (ScoreT)
*
65
60
55
50
45
40
35
30
High Risk psychosis patients*
Low Risk psychosis patients*
GAF
*
80
70
60
50
40
30
High Risk psychosis patients*
Low Risk psychosis patients*
Positive symptoms
*
16
14
12
10
8
6
4
High Risk psychosis patients*
Low Risk psychosis patients*

Figure 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Identification of miR-22-3p, miR-92a-3p, and miR-137 in peripheral blood as biomarker for schizophrenia. **MA JIE et al.** PSYCHIATRY RESEARCH. ELSEVIER IRELAND LTD, IE, 2018, vol. 265, 70-76
- Remington's Pharmaceutical Sciences. Mack Publishing Company, 1990, 1445